(19) Europäisches Patentamt / European Patent Office / Office européen des brevets

(11) **EP 3 658 690 B1**

(12) **EUROPEAN PATENT SPECIFICATION**

(45) Date of publication and mention
of the grant of the patent:
**19.03.2025 Bulletin 2025/12**

(21) Application number: **18769817.0**

(22) Date of filing: **23.07.2018**

(51) International Patent Classification (IPC):
**C12Q 1/6883** (2018.01)

(52) Cooperative Patent Classification (CPC):
(C-Sets available)
**C12Q 1/6883;** C12Q 2600/158 (Cont.)

(86) International application number:
**PCT/PL2018/000076**

(87) International publication number:
**WO 2019/022627 (31.01.2019 Gazette 2019/05)**

(54) **A METHOD FOR DETERMINING TISSUE INSULIN SENSITIVITY, A METHOD FOR IDENTIFYING INSULIN RESISTANCE AND/OR USE OF AN INSULIN SENSITIVITY INDEX**

VERFAHREN ZUR BESTIMMUNG DER GEWEBEINSULINEMPFINDLICHKEIT, VERFAHREN ZUR IDENTIFIZIERUNG DER INSULINRESISTENZ UND/ODER VERWENDUNG EINES INSULINEMPFINDLICHKEITSINDEXES

PROCEDE DE DÉTERMINATION DE LA SENSIBILITÉ A L'INSULINE TISSULAIRE, PROCEDE D'IDENTIFICATION DE LA RÉSISTANCE A L'INSULINE ET/OU UTILISATION D'UN INDICE DE SENSIBILITÉ A L'INSULINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2017 PL 42238717**

(43) Date of publication of application:
**03.06.2020 Bulletin 2020/23**

(73) Proprietor: **Uniwersytet Medyczny W Bialymstoku
15-089 Bialystok (PL)**

(72) Inventors:
- **STRACZKOWSKI, Marek
  15-225 Bialystok (PL)**
- **KARCZEWSKA-KUPCZEWSKA, Monika
  15-337 Bialystok (PL)**
- **KOBLOWSKA, Marta
  01-943 Warsaw (PL)**
- **IWANICKA-NOWICKA, Roksana
  01-038 Warsaw (PL)**
- **FOGTMAN, Anna
  42-224 Czestochowa (PL)**

(74) Representative: **Kawczynska, Marta Joanna
Polservice
Kancelaria Rzecznikow
Patentowych sp. z o.o.
ul. Bluszczanska 73
00-712 Warszawa (PL)**

(56) References cited:
EP-A1- 2 354 244      WO-A1-2010/096875
WO-A1-2011/019363     US-A1- 2003 092 028

- **TASSEVA GUERGANA ET AL: "Lack of phosphatidylethanolamineN-methyltransferase in mice does not promote fatty acid oxidation in skeletal muscle", BIOCHIMICA ET BIOPHYSICA ACTA (BBA) - MOLECULAR AND CELL BIOLOGY OF LIPIDS, ELSEVIER, AMSTERDAM, NL, vol. 1861, no. 2, 18 November 2015 (2015-11-18), pages 119 - 129, XP029352741, ISSN: 1388-1981, DOI: 10.1016/J.BBALIP.2015.11.008**

- **NING SHEN ET AL: "An Early Response Transcription Factor, Egr-1, Enhances Insulin Resistance in Type 2 Diabetes with Chronic Hyperinsulinism", JOURNAL OF BIOLOGICAL CHEMISTRY, vol. 286, no. 16, 22 April 2011 (2011-04-22), US, pages 14508 - 14515, XP055527412, ISSN: 0021-9258, DOI: 10.1074/ jbc.M110.190165**
- **"A GeneChip ? Human Genome Arrays", AFFYMETRIX DATASHEET, 1 January 2003 (2003-01-01), XP055182252**

(52) Cooperative Patent Classification (CPC): (Cont.)

C-Sets
**C12Q 1/6883, C12Q 2537/165**

## Description

## Technical Field

The invention as defined by the appended claims relates to an *in vitro* method for determining tissue insulin sensitivity in human subjects using the measurement of expression of selected genes in whole blood with the use of an insulin sensitivity index. The invention also relates to a method for identifying insulin resistance.

## Background Art

Decreased response of an organism to insulin, i.e. insulin resistance, is the most important factor in the pathogenesis of type 2 diabetes. Insulin resistance is also connected with a predisposition for dyslipidaemia, arterial hypertension, atherosclerosis, ischaemic heart disease, neurodegenerative diseases and many other illnesses. Although a relationship between insulin resistance and obesity is known, reduced tissue insulin sensitivity may also occur in people with normal body weight. Reduced tissue insulin sensitivity in the case of many diseases and disorders has been described in literature (see, for example, Reaven GM. Banting lecture 1988. "Role of insulin resistance in human disease", Diabetes 1988, 37: 1595-1607). Insulin resistance and chronic inflammatory response connected therewith plays a role, e.g., in the pathogenesis of atherosclerosis and cardiovascular diseases (see, for example, Fernandez-Real JM, Ricart W. "Insulin resistance and chronic cardiovascular inflammatory syndrome", Endocr Rev 2003, 24: 278-301, and in the publication of Nigro J, Osman N, Dart AM, Little PJ. Insulin resistance and atherosclerosis. Endocr Rev 2006, 27: 242-259). There has also been described the role of reduced insulin sensitivity in the brain aging process and cognitive impairment (see, for example, Roriz-Filho J, Sa-Roriz TM, Rosset I, Camozzato AL, Santos AC, Chaves ML, Moriguti JC, Roriz-Cruz M. (Pre) diabetes, brain aging, and cognition. Biochim Biophys Acta 2009, 1792: 432-443).

Early diagnosis of insulin resistance may therefore be essential for taking prophylactic measures in order to prevent diseases and disorders connected therewith. Methods for the assessment of tissue insulin sensitivity are known. There are available review articles concerning methods for the measurement of tissue insulin sensitivity.

At present, the "gold standard" among the methods for the *in vivo* assessment of tissue insulin sensitivity is represented by hyperinsulinemic-euglycemic clamp technique, which was introduced by DeFronzo et al. This method was described in detail in an article by DeFronzo RA, Tobin JD, Andres R. "Glucose clamp technique: a method for quantifying insulin secretion and resistance.", Am J Physiol 1979, 237: E214-E223. Since the time of publishing the article, the clamp technique has been widely used in studies including tests in people; however, the practical use of this technique is limited to scientific research carried out in specialized centres. A test by clamp technique requires two venipunctures, at least 2-hour continuous infusion of insulin and variable controlled infusion of glucose, and performing blood draw every 5 minutes. It requires using specialized medical equipment, employing qualified medical personnel and is troublesome and inconvenient for the person subjected to the test.

The available literature concerning methods for assessing tissue insulin sensitivity highlights limitations connected with tests by the clamp technique, as well as other methods used for this purpose, such as insulin suppression test which requires 3-hour infusion of glucose, insulin and samatostatin or its analogue - octreotide (see, for example, Muniyappa R, Madan R, Quon MJ. "Assessing insulin sensitivity and resistance in humans", in: De Groot LJ, Chrousos G, Dungan K, Feingold KR, Grossman A, Hershman JM, Koch C, Korbonits M, McLachlan R, New M, Purnell J, Rebar R, Singer F, Vinik A, ed. Endotext [Internet]. South Dartmouth (MA): MDText.com, Inc.; 2000-. ; Antuna-Puente B, Disse E, Rabasa-Lhoret R, Laville M, Capeau J, Bastard JP. "How can we measure insulin sensitivity/resistance?", Diabetes Metab 2011, 37: 179-188; 9; Wallace TM, Matthews DR., "The assessment of insulin resistance in man.", Diabet Med 2002, 19: 527-534; Ferrannini E, Mari A. "How to measure insulin sensitivity." J Hypertens 1998, 16: 895-906). The limitations of currently available diagnostic methods described in the literature are primarily connected with the fact that these tests are time-consuming, laborious and expensive, and they require specialized equipment and qualified personnel. That is why, neither the clamp technique nor the insulin suppression test are deemed to be capable of application in epidemiological studies, large clinical trials and in everyday medical practice.

There have also been described other dynamic tests for assessing tissue insulin sensitivity, such as the intravenous glucose tolerance test with the minimal model analysis. However, it also requires intravenous infusion and multiple blood draws. Additionally, while being almost equally laborious, it is not as precise as the clamp technique.

The known methods of determining tissue insulin sensitivity include indices based on the oral glucose tolerance test. It is a 2-hour, standard 75-gram glucose load test used in clinical practice, in which glucose tolerance is assessed. One exemplary tissue insulin sensitivity index developed on the basis of the oral glucose tolerance test is the Matsuda Index (Matsuda M. "Measuring and estimating insulin resistance in clinical research setting.", Nutr Metab Cardiovasc Dis 2010, 20: 79-86). To calculate this index, it is necessary to determine the concentration of glucose and insulin in the blood in 0, 30th, 60th, 90th and 120th minute of the glucose tolerance test, which is rather time-consuming and requires multiple blood draws. It must be emphasized that glucose tolerance is affected by not only tissue insulin sensitivity but also insulin secretion, insulin absorption rate, incretin effect, suppression of glucagon level and many other factors. The oral glucose tolerance test shows also low reproducibility (see, for example, Ko GT, Chan JC, Woo J, Lau E, Yeung VT, Chow CC, Cockram CS. "The reproducibility and usefulness of the oral glucose tolerance test in screening for diabetes and other cardiovascular risk factors." Ann Clin Biochem 1998; 35: 62-67; Libman IM, Barinas-Mitchell E, Bartucci A, Robertson R, Arslanian S. "Reproducibility of the Oral Glucose Tolerance Test in Overweight Children." J Clin Endocrinol Metab 2008; 93: 4231-4237). The measurement of insulin sensitivity based on the oral glucose tolerance test has failed to find application in general population or diagnostic and epidemiological studies, or in everyday medical practice.

There have also been developed simple indices that were intended to reflect tissue insulin sensitivity, such as HOMA-IR (Homeostasis Model Assessment - Insulin Resistance) or QUICKI (Quantitative Insulin Sensitivity Check Index), involving the measurement of fasting blood glucose and insulin concentrations. These indices also have some limitations resulting, among others, from the fact that they primarily reflect hepatic insulin sensitivity/resistance, whereas, actually, in the predisposition for/the development of a disorder connected with reduced tissue insulin sensitivity, such as type 2 diabetes, the most import role is played by peripheral insulin resistance, for which skeletal muscles are primarily responsible (and which is measured in the clamp technique test). It should be emphasized that insulin is secreted in a pulsatile manner and glucose concentration in the blood is controlled by not only insulin but also many other hormones, e.g., glucagon. Individual fluctuations in insulinaemina are also affected by many other factors, e.g., stress or physical activity. Although such indices as HOMA-IR and QUICKI generally show good correlation with results obtained using the clamp technique, when human subjects with a marked insulin resistance and/or glucose intolerance are taken into account, this relation is considerably weaker in general population. For these reasons HOMA-IR and QUICKI indices have some limitations in indentifying human subjects with insulin resistance in general population (Ruige JB, Mertens IL, Bartholomeeusen E, Dirinck E, Ferrannini E, Van Gaal LF., "Fasting-based estimates of insulin sensitivity in overweight and obesity: a critical appraisal.", Obesity 2006, 14: 1250-1256; Malita FM, Karelis AD, St-Pierre DH, Garrel D, Bastard JP, Tardif A, Prud'homme D, Rabasa-Lhoret R., "Surrogate indexes vs. euglycaemic-hyperinsulinemic clamp as an indicator of insulin resistance and cardiovascular risk factors in overweight and obese postmenopausal women.", Diabetes Metab 2006, 32: 251-255).

Considering the fact that dynamic tests are complicated, time-consuming and laborious, hardly reproducible, and indices based on the determination of fasting blood glucose and insulin are excessively simplified, and to a lesser extent assess systemic insulin resistance, at present, there is no ideal simple index assessing tissue insulin sensitivity in clinical and population studies and in everyday medical practice.

## Object of the Invention

The object of the present invention is to provide simple and reliable means and methods which reflect tissue insulin sensitivity and enable the identification, of insulin resistance in general population, and which simultaneously ensure repeatable results with high sensitivity and specificity. The object of the present invention is therefore to provide new, effective tools for determining tissue insulin sensitivity, ensuring repeatable results with high sensitivity and specificity, in a quick, reliable and cost-effective way, which area suitable for routine applications in general population, in epidemiological studies, large clinical trials and in everyday medical practice, including the assessment of tissue insulin sensitivity, identification of insulin resistance The object of the present invention is also to provide new, effective tools for *in vitro* determining tissue insulin sensitivity and identifying insulin resistance in a manner that is convenient for the patient, which minimizes his discomfort and pain connected with long-lasting tests, multiple insertions of needles and multiple blood draws. The above objects have been realized by the invention described below and defined in the patent claims.

## Summary of the Invention

The subject matter of the present invention relates to a method for determining tissue insulin sensitivity in a human subject, consisting in that

a) RNA is isolated from a whole blood sample drawn from a human subject and an expression of the following genes is measured: KLF9, PEMT and EGR1;
b) an insulin sensitivity index is determined on the basis of obtained results of the measurements specified in a) using the following equation:

Insulin sensitivity index = 7.2109 - log2 KLF9 x 1.8717 + log2 PEMT x 1.2903 + log2 EGR1 x 0.1145, wherein

KLF9 is the value of measured expression of KLF9 gene,

PEMT is the value of measured expression of PEMT gene,

EGR1 is the value of measured expression of EGR1 gene;

wherein the obtained index value of 6.3 or less indicates a reduced tissue insulin sensitivity,

and wherein lower result corresponds to lower tissue insulin sensitivity and a higher result corresponds to higher tissue insulin sensitivity (also within the range below and above the cut-off point)

Preferably, in the methods according to the invention, in step a) RNA is isolated and the expression of the indicated genes is measured using a reverse transcription method and Real Time PCR method.

More preferably, in the methods according to the invention, in the Real Time PCR method the following primers are used: for KLF9 gene - a forward primer having a sequence No. 1 (5'- CTCCGAAAAGAGGCACAAGT) and a reverse primer having a sequence No. 2 (5'- CGGGAGAACTTTTTAAGGCAGT), for PEMT gene - a forward primer having a sequence No. 3 (5'-GGCCTCGGCAATATTGATT) and a reverse primer having a sequence No. 4 (5'-GTCCACGTAG CCCAGCAG), for EGR1 gene - a forward primer having a sequence No. 5 (5'-AGCACCTGACCGCAGAGT) and a reverse primer having a sequence No. 6 (5'- GGCAGTCGAGTGGTTTGG).

More preferably, in the above methods according to invention, mRNA expression of a reference gene, in particular GAPDH, is also measured.

Even more preferably, in the methods according to the invention, in the Real Time PCR method the following primers are used for the reference gene GAPDH: a forward primer having a sequence No. 7 (5'-CTCTGCTCCTCCT GTTCGAC) and a reverse primer having a sequence No. 8 (5'- ACGACCAAATCCGTTGACTC).

In the above methods the human subject from whom the sample is derived is preferably selected from a group comprising: a person with normal weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardiovascular diseases, and a person before bariatric surgery and/or after such surgery.

The subject matter of the invention further relates to an insulin sensitivity index defined by the following equation:

$$\text{Insulin sensitivity index} = 7.2109 - \log_2 KLF9 \times 1.8717 + \log_2 PEMT \times 1.2903 + \log_2 EGR1 \times 0.1145,$$

wherein

KLF9 is the value of measured expression of KLF9 gene,

PEMT is the value of measured expression of PEMT gene,

EGR1 is the value of measured expression of EGR1 gene;

Preferably mRNA expression of the indicated genes is measured by a reverse transcription method and Real Time PCR method.

More preferably, in the Real Time PCR method, the following primers are used: for KLF9 gene - a forward primer having a sequence No. 1 (5'-CTCCGAAAAGAGGCACAAGT) and a reverse primer having a sequence No. 2 (5'- CG GGAGAACTTTTTAAGGCAGT), for PEMT gene - a forward primer having a sequence No. 3 (5'- GGCCTCGGCAATA TTGATT) and a reverse primer having a sequence No. 4 (5'- GTCCACGTAGCCCAGCAG), for EGR1 gene - a forward primer having a sequence No. 5 (5'- AGCACCTGACCGCAGAGT) and a reverse primer having a sequence No. 6 (5'- GGCAGTCGAGTGGTTTGG).

More preferably, mRNA expression of a reference gene, in particular GAPDH, is also measured.

Even more preferably, in the Real Time PCR method, the following primers are used for the reference gene GAPDH: a forward primer having a sequence No. 7 (5'- CTCTGCTCCTCCTGTTCGAC) and a reverse primer having a sequence No. 8 (5'- ACGACCAAATCCGTTGACTC).

The insulin sensitivity index for uses as defined above is more preferably applied in a human subject selected from a group comprising: a person with normal weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardiovascular diseases, and a person before bariatric surgery and/or after such surgery.

The subject matter of the invention further relates to the use of an insulin sensitivity index defined by the following equation:

Insulin sensitivity index = 7.2109 - log2 KLF9 x 1.8717 + log2 PEMT x 1.2903 + log2 EGR1 x 0.1145, wherein

KLF9 is the value of measured expression of KLF9 gene,
PEMT is the value of measured expression of PEMT gene,
EGR1 is the value of measured expression of EGR1 gene;
for monitoring tissue insulin sensitivity, in particular in type 2 diabetes, wherein the obtained index value of 6.3 or less indicates a reduced tissue insulin sensitivity.

Preferably, in such use, mRNA expression of the indicated genes is measured by a reverse transcription method and Real Time PCR method.

More preferably, in the Real Time PCR, the following primers are used: for KLF9 gene - a forward primer having a sequence No. 1 (5'-CTCCGAAAAGAGGCACAAGT) and a reverse primer having a sequence No. 2 (5'- CGGGAGAA CTTTTTAAGGCAGT), for PEMT gene - a forward primer having a sequence No. 3 (5'- GGCCTCGGCAATATTGATT) and a reverse primer having a sequence No. 4 (5'- GTCCACGTAGCCCAGCAG), for EGR1 gene - a forward primer having a sequence No. 5 (5'- AGCACCTGACCGCAGAGT) and a reverse primer having a sequence No. 6 (5'- GGCAGTCGA GTGGTTTGG).

More preferably, mRNA expression of a reference gene, in particular GAPDH, is also measured.

Even more preferably, in the Real Time PCR method, the following primers are used for the reference gene GAPDH: a forward primer having a sequence No. 7 (5'- CTCTGCTCCTCCTGTTCGAC) and a reverse primer having a sequence No. 8 (5'- ACGACCAAATCCGTTGACTC).

## Detailed Description of the Invention

The present inventors have developed a new method for determining *in vitro* tissue insulin sensitivity using the measurement of expression of the selected genes in a sample obtained from one blood draw from the examined human subject, such a whole blood sample. More specifically, the method for determining tissue insulin sensitivity according to the invention requires determining the expression of the following genes: KLF9, PEMT and EGR in a blood sample obtained from one blood draw from the patient, preferably mRA expression of the above-indicated genes. For this purpose it is required to use any standard methods, reagents, kits and apparatuses, commonly known and used in the field of the procedures for the preparation of biological material, RNA isolation and the measurement of gene expression, including mRNA expression. The results of the above-mentioned measurements are used for determining tissue insulin sensitivity with use of the insulin sensitivity index developed by the present inventors. The inventions according to the present application also make it possible to identify insulin resistance in the examined human subject.

The solutions according to the invention make it possible to precisely determine tissue insulin sensitivity and/or insulin resistance in a quick, reliable, repeatable and cost-effective way. The results obtained from the method for determining tissue insulin sensitivity according to the invention and the method for identifying insulin resistance are reliable and repeatable also due to fact that they are independent of insulinaemina fluctuations caused by stress or physical activity. Thus, the solutions according to the invention are perfectly suitable for use in general population.

The inventions as defined in the patent claims are suitable for use in general population, in particular in the following human subjects: a person with normal weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardio-vascular diseases. Moreover, the inventions are applicable in human subjects scheduled for bariatric surgery in whom tissue insulin sensitivity or insulin resistance may be a deciding element in the choice of an appropriate bariatric surgery, and human subjects after such surgery in whose case determining tissue insulin sensitivity or insulin resistance may be a deciding element in the choice of appropriate further therapy. The methods according to the invention are not troublesome and inconvenient for a human subject subjected to testing, they minimize his/her pain and the feeling of discomfort connected with a long-lasting tests and multiple insertions of needles required in the methods used so far in the field of the present invention.

The methods according to the invention have been developed by referring the results of determination of a panel of standard laboratory tests and protein concentrations in serum to the measurement of tissue insulin sensitivity by an hyperinsulinemic-euglycemic clamp method, which is the 'gold standard' in the assessment of insulin action *in vivo*.

Definitions

The technical and scientific terms used in the present description have standard meanings used in the field of the present invention. The definitions below have been provided to make it easier for the reader to carry out the present invention.

The term "insulin sensitivity" in the context of the present invention relates to the ability to properly respond to insulin, which is manifested, first of all, in an increase in skeletal muscle glucose uptake, inhibition of lipolysis and hepatic glucose production. Insulin sensitivity may be measured, e.g., by a hyperinsulinemic-euglycemic clamp method (in short, the clamp technique), which is currently the 'gold standard' in determining tissue insulin sensitivity. In this technique, the value of insulin-dependent glucose uptake (M value, hereafter also referred to as variable M) may be expressed in mg/kg of Fat-Free-Mass (FFM)/min.

The term "reduced insulin sensitivity" or "insulin resistance" in the context of the present invention means decreased response of an organism to endogenous or exogenous insulin as regards the metabolism of carbohydrates, lipids and proteins. In the hyperinsulinemic-euglycemic clamp method, insulin resistance is usually assumed when the value is less than 6 mg/kg FFM/min. In the context of the present invention, insulin resistance is assumed when the value determined using the insulin sensitivity index defined according to the present invention is 6.3 or less. Furthermore, it is necessary to point out that according to the present invention, a lower result corresponds to lower insulin sensitivity (also within the range below the cut-off point), and a higher result corresponds to higher tissue insulin sensitivity (also within the range above the cut-off point). Thus, the lower the value of the determined insulin sensitivity index, the lower tissue insulin sensitivity (also within the range of the cut-off point), which indicates the presence of insulin resistance in the examined human subject. Identifying insulin resistance makes it possible to diagnose the examined person with a predisposition for a disorder connected with insulin resistance. On the other hand, a higher obtained value of the insulin sensitivity index corresponds to higher tissue insulin sensitivity (also within the range above the cut-off point).

The symptoms of insulin resistance are abnormally elevated blood insulin and/or glucose concentrations. Insulin resistance predisposes a human subject to the development of a number of abnormalities or disorders, including impaired glucose tolerance, and subsequently type 2 diabetes, atherogenic dyslipidaemia, i.e. hypertriglyceridemia, lowered concentration of high density lipoprotein (HDL) cholesterol in the blood and the presence of small low density lipoproteins (LDL), hyperuricemia, reduced plasma fibrinolytic activity, arterial hypertension, atherosclerosis, cardiovascular diseases (Roriz-Filho, J. et al. Biochim Biophys Acta. 1792: 432-443, 2009).

The term "disorder connected with insulin resistance" relates to a disorder in which the response to insulin action is reduced; in other words, insulin sensitivity is reduced. Disorders connected with insulin resistance, i.e. reduced insulin sensitivity, comprise, among others, overweight, obesity, impaired glucose tolerance, dyslipidemia, fatty liver, metabolic syndrome, polycystic ovary syndrome, cardiovascular diseases, including arterial hypertension, atherosclerosis, ischaemic heart disease, cerebral stroke; neurodegenerative diseases, certain neoplasms, and in particular type 2 diabetes.

The term "measurement of gene expression" means herein any method for measuring gene expression in a biological sample taken from a human subject, such as a whole blood sample. In the context of the present invention, the measurement of gene expression is preferably understood as mRNA expression measurement using a reverse transcription method and Real Time polymerase chain reaction (real-time PCR or qPCR), also called a quantitative PCR, Real Time quantitative PCR, qPCR RQ-PCR, RTQ-PCR. These names are synonyms denoting a modified polymerase chain reaction that makes it possible to monitor, on a current basis, an increase in the amount of product during the reaction, thanks to the addition of fluorescent compounds to the reaction mixture. Emission of fluorescence depends on the kinetics of the increase in the number of DNA particles. The conditions for performing a Real Time PCR are known. However, in order to measure gene expression, other methods may also be used, such as digital PCR, NanoString and methods for large-scale microarray analysis and sequencing, such as sequencing of DNA (for example, cDNA generated in the RNA reverse transcription) and RNA. Sequencing may be performed using any known method or technique. Such sequencing methods comprise: New Generation Sequencing, high through output sequencing, pyrosequencing, sequencing by ligation, sequencing by synthesis, sequencing by hybridization, RNA-seq technique, single-molecule sequencing by synthesis(SMSS), semiconductor sequencing, nanopore sequencing, single molecule real time sequencing (SMRT), massively parallel sequencing.

In the case of measurement methods used to determine gene expression which have different characteristics than the Real Time PCR method, for example, different sensitivity and/or reference interval of obtained measurement values, to the calculation of the insulin sensitivity index according to the present invention, it may be required to appropriately correct the relevant index calculations, taking into account the value intervals of expression measurements in the applied method.

The term "primer" used herein means a short segment of a nucleic acid sequence which forms base pairs with the complete template and has a free 3'-hydroxy group which is used as the origin of replication for template strands. DNA synthesis may start with the participation of primers in the presence of a polymerase factor, for example, DNA polymerase or reverse transcriptase in suitable conditions of buffer solutions and temperature and in the presence of four different nucleoside triphosphates. These conditions, buffers and enzymes are known and commonly used by persons skilled in this art. In this invention any DNA polymerases and reverse transcriptases that are suitable for use in a reverse transcription reaction and PCR may be used. Specific conditions of a reverse transcription reaction and PCR can be selected in a routine way by persons skilled in this art.

KLF9 gene, in context of the present invention, means a gene encoding a protein - Kruppel-like factor 9 which is a transcription factor with zinc finger motif, binding elements of GC cassette located in the promoter region. KLF 9 controls a number of cellular processes, e.g., cell differentiation. It also interacts with the progesterone receptor. The level of expression of this gene, in particular mRNA, is used for calculating the insulin sensitivity index developed by the present inventors, which is defined by the equation described according to the invention.

PEMT gene means a gene encoding a protein - phosphatidylethanolamine N-methyltransferase, converting phosphatidylethanolamine into phosphatidylcholine. It has been shown in experimental studies that this enzyme participates in the development of obesity, fatty liver and cardiovascular diseases. The level of expression of this gene, in particular mRNA, is used for calculating the insulin sensitivity index developed by the present inventors, which is defined by the equation described according to the invention.

EGR1 gene means a gene encoding a nuclear protein - early growth response 1, which functions as a transcription regulator, the target genes of which are required for differentiation and mitogenesis. Data indicate that EGR1 functions as a tumour suppressor. It also regulates activity and plasticity of neurons. The level of expression of this gene, in particular mRNA, is used for calculating the insulin sensitivity index developed by the present inventors, which is defined by the equation described according to the invention.

A reference gene, in the context of the present invention, means an endogenous gene which is characterized by relatively constant expression in various experimental conditions and which is used as an internal control of PCR reaction. For this purpose, housekeeping genes, such as GAPDH, without limitation thereto, are used.

GAPDH gene means a housekeeping gene that encodes a housekeeping metabolism protein - glyceraldehyde-3-phosphate dehydrogenase. This enzyme catalyses reactions of the glycolysis process - conversion of glyceraldehyde-3-phosphate into 1,3-bisphosphoglycerate. Thus, it plays an important role in obtaining energy from carbohydrates by cells. GAPDH gene is commonly used a reference gene or an internal control in the quantitative determination of gene expression using a method of Real Time polymerase chain reaction (PCR).

The term "sensitivity" means the ratio of true positives to the sum of true positives and false negatives obtained in a given test. Sensitivity determines the ability of a test to detect human subjects that are diseased or predisposed for the development of a disease. Sensitivity is defined by the following formula:

$$\text{SENSITIVITY} = (\text{PD} / \text{PD} + \text{FU}) \text{ X } 100,$$

wherein PD denotes true positives, and FU denotes false negatives.

In the context of the of the present invention, the term "sensitivity" used in relation to a diagnostics method or test means an index that shows what fraction of all examined subjects with insulin sensitivity lower than 6 mg/kg FFM/min in the test using the clamp technique is correctly identified by a decision criterion based on given cut-off value. The cut-off value is calculated as the ratio of the number of human subjects in whom a method or a test truly indicates insulin resistance to all observed human subjects in whom insulin resistance occurred. In the test using the insulin sensitivity index according to the invention, said value is 6.3.

The term "specificity" means the ratio of true negatives to the sum of true negatives and false positives obtained in a given test. It determines the ability of the test to detect human subjects that are healthy or do not have a predisposition for the development of disease or, in other words, to correctly exclude a disease or a predisposition thereto, and it relates only to the population of healthy human subjects. Specificity is defined by the following formula:

$$\text{SPECIFICITY} = (\text{PU}/\text{PU} + \text{FD}) \text{ X } 100\%,$$

wherein PU denotes true negatives, and FD denotes false positives.

In the context of the of the present invention, the term "specificity" means an index that shows what fraction of human subjects in whom insulin resistance does not occur is correctly identified by a decision criterion based on given a cut-off value in a given test. The cut-off value is calculated as the ratio of human subjects with a true negative result of the test to all observed human subjects in whom insulin resistance was higher than or equal to 6 mg/kg FFM/min in the test using the clamp technique. In the test using the insulin sensitivity index according to the invention, said value is 6.3.

The term "diagnostic power" means its accuracy. One very common index of diagnostics power is the Area Under Curve (AUC) of Receiver Operating Characteristic (ROC). The value of AUC index falls within the rage from 0 to 1; the higher the value, the better the diagnostic power of a diagnostic method or test. In the test using the insulin sensitivity index according to the invention the value is 0.82.

At present, an accurate assessment of tissue insulin sensitivity is difficult and laborious - it requires the use of at least 2-hour testing by the metabolic clamp technique, with two venipunctures and infusion of insulin and glucose. Considering the fact that it is difficult and laborious, this method is not suitable for use on the population level for routine diagnostic tests in general population. The available indices based on the fasting blood concentration of glucose and insulin are not sufficiently sensitive to diagnose insulin resistance in its early stages, e.g., in young generally healthy human subjects. On the other hand, indices based on glucose and insulin concentrations during an oral glucose tolerance test show low reproducibility (in some test it is only about 30%).

The subject matter of the present invention relates to a method for determining, *in vitro,* tissue insulin sensitivity in a human subject, consisting in that

a) RNA is isolated from a whole blood sample drawn from a human subject and the expression of the following genes is measured: KLF9, PEMT and EGR1;

b) an insulin sensitivity index is determined on the basis of obtained results of the measurements specified in a) using the following equation:

Insulin sensitivity index = 7.2109 - log2 KLF9 x 1.8717 + log2 PEMT x 1.2903 + log2 EGR1 x 0.1145, wherein

KLF9 is the value of the measured expression of KLF9 gene,
PEMT is the value of the measured expression of PEMT gene,
EGR1 is the value of measured expression of EGR1 gene.

Calculating the insulin sensitivity index on the basis of the above-described measurements makes it possible to determine a precise and reliable tissue insulin sensitivity in the human subject being tested.

The insulin sensitivity index value of 6.3 or less obtained in the method for identifying insulin resistance and/or a predisposition for a disorder connected with insulin resistance indicates reduced tissue insulin sensitivity, i.e. insulin resistance. A lower result corresponds to lower tissue insulin sensitivity (also within the range below the cut-off point), and a higher result corresponds to higher tissue insulin sensitivity (also within the range above the cut-off point). Determining insulin resistance makes it possible then to diagnose a predisposition for the development of a disorder connected with insulin resistance in the human subject being tested, as described above.

A new feature of the present invention consists in a unique combination of the results of measurements of gene expression in whole blood so that they predict tissue insulin sensitivity in a given person in the best way. To carry out the methods according to the invention, only one fasting blood draw is required. According to the invention, a specific numerical value is obtained which may be interpreted both as the diagnosis of insulin resistance or the lack thereof (insulin resistance - YES/NO depending on the relation to the cut-off point) and a continuous variable, a higher value of which reflects higher tissue insulin sensitivity.

The solutions according to the invention have been developed with the use of complex regression models (ridge, lasso, random forest, supervised principal components regression) as an optimal model of determining insulin resistance Compared to the known insulin sensitivity tests based, e.g., on a 2-hour oral glucose tolerance test, only one blood draw is required. It should be emphasized that the methods according to the invention are repeatable, specific and sensitive. Moreover, they are characterized by high diagnostic power. The sensitivity of the methods according to the present invention is 80%, the specificity is 78%, whereas the diagnostic power is 0.82.

Insulin resistance can be detected in young human subjects many years before the development of disorders and/or diseases connected therewith. Thus, the development of a simple index for assessing the level or degree of tissue insulin sensitivity and insulin resistance finds a significant application in the prevention of type 2 diabetes and other disorders and diseases connected with insulin resistance. The solutions according to the invention are perfectly suitable for this purpose, in particular for the use in general population.

When developing the present disclosure 150 young healthy volunteers with different body mass index were subjected to examination and their insulin sensitivity was measured using the metabolic clamp technique which is 'the gold standard' in the assessment of insulin action *in vivo*. Part of the examination involved a detailed clinical characteristics of the examined human subjects and then complex regression models were used in order to detect an array of factors determining tissue insulin sensitivity in the optimal way. This made it possible to develop methods, uses and indices for the uses, in which the results of measurements of the expression of selected genes are used. They make it possible to obtain a specific numerical value and refer it to the recommended values. This allows to assess tissue insulin sensitivity in the examined human subject in a quick and reliable way, on the basis of a blood sample taken from one draw. This makes it possible to carry out the method for identifying insulin resistance It should be emphasized that the solutions according to the invention are suitable for use in general population, in order to identify insulin resistance or decreased insulin sensitivity, and in particular they can be used in persons with a positive family history of diabetes, in obese persons, persons with arterial hypertension, cardio-vascular disease, lipid disorders, in women with polycystic ovary syndrome, in women who had gestational diabetes or gave birth to a baby having over 4 kg body weight and persons before bariatric surgery and/or after such surgery.

Determining tissue insulin sensitivity and indentifying insulin resistance in accordance with the present invention requires only one whole blood draw and the measurement of expression of the selected genes. The measurement of gene expression is preferably carried out according to the present invention by isolating RNA and measuring mRNA expression of the above-indicated genes using a reverse transcription method and Real Time PCR method. The conditions for carrying out such reactions are known to persons skilled in this art and they have been described in the literature (see, for example, Holland PM, Abramson RD, Watson R, Gelfand DH. Detection of specific polymerase chain reaction product by utilizing the 5' → 3' exonuclease activity of Thermus aquaticus DNA polymerase. Proc Natl Acad Sci USA 1991, 88, 7276-7280; Gibson UEM, Heid CA, Williams PM. A Novel Method for Real Time Quantitative RT-PCR. Genome Res 1996, 6, 995-1001; Bustin SA. Absolute quantification of mRNA using real time reverse transcription polymerase chain reaction assays. J Mol Endocrinol 2000, 25, 169-193). Exemplary, preferable methods and conditions for RNA isolation and for carrying out a reverse transcription reaction and Real Time PCR are given below in the Examples.

However, to measure the expression of the herein indicated genes, various methods may be used, such as a digital PCR, NanoString and methods for large-scale microarray analysis and sequencing, such as sequencing of DNA (for example, cDNA generated in the RNA reverse transcription reaction) and RNA. Sequencing may be performed using any known method or technique. Such sequencing methods comprise: New Generation Sequencing, high through output sequencing, pyrosequencing, sequencing by ligation, sequencing by synthesis, sequencing by hybridization, RNA-seq technique, single-molecule sequencing by synthesis (SMSS), semiconductor sequencing, nanopore sequencing, single molecule real time sequencing (SMRT), massively parallel sequencing. For a detailed description of the above methods for the measurement of gene expression which may be used according to the present invention see, for example, Cao L at al. "Advances in Digital Polymerase Chain Reaction (dPCR) and Its Emerging Biomedical Applications", Biosens Bioelectron 2017, 90, 459-474; NanoString Technologies, nCounter® Gene Expression "Strategies for Successful Gene Expression Assays", Tech Note, 2015; Veldman-Jones MH et al. "Evaluating Robustness and Sensitivity of the NanoString Technologies nCounter Platform to Enable Multiplexed Gene Expression Analysis of Clinical Samples" Cancer Res 2015, 75 (13): 2587-93; Sabour L et al. "Clinical Applications of Next-Generation Sequencing in Cancer Diagnosis" Pathol Oncol Res 2016, 23 (2), 225-234; Mardis ER "DNA sequencing technologies: 2006-2016" Nat Protoc. 2017; 12 (2): 213-218; Kamps R at al. "Next-Generation Sequencing in Oncology: Genetic Diagnosis, Risk Prediction and Cancer Classification" Int J Mol Sci 2017; 18 (2): 308; Morozova O et al. "Applications of new sequencing technologies for transcriptome analysis" Annu Rev Genomics Hum Genet 2009; 10: 135-51; Yadav NK "Next Generation Sequencing: Potential and Application in Drug Discovery" Scientific World Journal 2014; Wang J, Song Y "Single cell sequencing: a distinct new field" Clin Transl Med 2017; 6 (1):10; Cuevas-Diaz Duran R, Wei H, Wu JQ "Single-cell RNA-sequencing of the brain" Clin Transl Med 2017; 6 (1): 20; Garrido-Cardenas JA, Garcia-Maroto F, Alvarez-Bermejo JA, Manzano-Agugliaro F "DNA Sequencing Sensors: An Overview" Sensors 2017, 17, 588; Gupta AK, Gupta UD, "Next Generation Sequencing and Its Applications", Animal Biotechnology Models in Discovery and Translation, Chapter 19, 2014; Harrington CT, Lin EI, Olson MT, Eshleman JR "Fundamentals of pyrosequencing" Arch Pathol Lab Med 2013;137(9):1296-303 and Hrdlickova R, Toloue M, Tian B "RNA-Seq methods for transcriptome analysis" Wiley Interdiscip Rev RNA 2017; 8 (1).

In the case of measurement methods used to determine gene expression which have different characteristics than the Real Time PCR method, for example, different sensitivity and/or reference interval of obtained measurement values, to the calculation of the insulin sensitivity index according to the present invention, it may be required to appropriately correct the relevant index calculations, taking into account the value intervals of expression measurements in the applied method.

It is also required to use standard, generally known procedures for the preparation of biological material, isolation of nucleic acids, including RNA, and for the measurement of gene expression, including mRNA, with the use of commonly known methods and commercially available apparatuses and kits. The results of the above measurements obtained herein are used for determining insulin sensitivity or insulin resistance with the use of the insulin sensitivity index developed by the present inventors and defined by the following equation:

Insulin sensitivity index = 7.2109 - log2 KLF9 x 1.8717 + log2 PEMT x 1.2903 + log2 EGR1 x 0.1145, wherein

KLF9 is the value of the measured expression of KLF9 gene,
PEMT is the value of the measured expression of PEMT gene,
EGR1 is the value of measured expression of EGR1 gene.

When interpreting the obtained result, it is recommended to also take into account the absolute value of the formula, as parameter reflecting the result of the clamp test, i.e. a lower result corresponds to lower tissue insulin sensitivity (also within the range below the cut-off point., and a higher result - to higher tissue insulin sensitivity (also within the range above the cut-off point). When the obtained value of the insulin sensitivity index is 6.3 or less, insulin resistance is identified.

If the measured value used to calculate the insulin sensitivity index as described herein is expressed in other units or reference intervals than those obtained using the reverse transcription method and Real Time PCR method, it must be converted accordingly.

Table 1 presents reference interval values for gene expression obtained preferably according to the invention.

Table 1. Means $\pm$ S.D. and gene expression value intervals used in the reverse transcription and Real Time PCR method.

| Gene | Mean | S.D. | Minimum | Maximum |
|------|------|------|---------|---------|
| KLF9 | 0.0368 | 0.0157 | 0.0193 | 0.1256 |
| PEMT | 0.0093 | 0.0056 | 0.0033 | 0.0431 |
| EGR1 | 0.0106 | 0.0087 | 0.0019 | 0.0507 |

In the case of the measurement methods used to determine the parameters necessary to calculate the insulin sensitivity index according to the present invention having a different range of obtained measurement values compared to those indicated above, it is possible to correct the calculations, i.e. if the mean value of a given parameter significantly differs from the reference interval, the multiplier should be corrected accordingly, i.e. if the mean value of the interval is five times higher, the multiplier of the given parameter should be reduced five times.

The sensitivity of the methods according to the present invention is 80%, and the specificity is 78%. The area under the Receiver Operating Characteristic (ROC) curve, i.e. the diagnostic power, is 0.82.

Experimental verification

The methods according to the invention were tested in a statistical analysis in the so-called learning sample and testing sample. Then, they were verified in samples derived from earlier studies done by the present inventors, which covered 400 human subjects in whom insulin sensitivity was measured by the metabolic clamp method. The index was successfully verified as a biomarker of reduced tissue insulin sensitivity, i.e. insulin resistance, in groups of human subjects with normal body weight, overweight, and obesity, in human subjects with impaired glucose tolerance, and in women with polycystic ovary syndrome.

The studies were carried out in a group of 150 young healthy volunteers with different body mass index in whom insulin sensitivity was measured using the metabolic clamp technique which represents 'the gold standard' in the assessment of insulin action *in vivo.* Part of the studies involved detailed clinical characteristics of the examined human subjects, the assessment of basis laboratory parameters and the measurement of the serum concentration of a panel of proteins.

Then, in order to detect an array of factors which determines tissue insulin sensitivity in the optimal way, complex regression models were constructed: the linear model with regularization (ridge and lasso), the supervised principal components model, and the random forest model. The randomly selected learning sample and testing sample were used in the analyses. The result of the clamp test, determining tissue insulin sensitivity, was subjected to categorization with the value of 6 mg/kg FFM/min adopted as the cut-off point. The inventors resigned from the division of the quality variable (tissue insulin sensitivity) according to the median and tertiles in the examined population due to the relationship of these values with the examined sample. Regression models were constructed for all examined variables and for the individual areas of variables (clinical characteristics, biochemical characteristics, gene expression in whole blood, gene expression in tissues, protein concentrations in blood). It was observed that the areas of variables concerning the clinical characteristics and gene expression make it possible to predict insulin sensitivity independently of each other. Additionally, the whole area of the clinical characteristics, comprising also measurements obtained during the oral glucose tolerance test, as well as the same area limited to the results obtained in fasting tests only were analysed.

The sensitivity and specificity of a given test was taken into account. Sensitivity is an index that indicates what fraction of all the examined subjects having less than 6 insulin sensitivity value is identified by the decision criterion based on the given cut-off value. Sensitivity is calculated as the proportion of the number of human subjects in whom the test truly indicates insulin resistance to all observed human subjects in whom it occurred. Specificity is an index that indicates what fraction on patients in whom insulin resistance does not occur is correctly identified by the decision criterion based on given cut-off value. It is calculated as the proportion of the number of human subjects with truly negative result of the test to all observed human subjects in whom insulin sensitivity was greater than or equal to 6.

Statistical analysis

The statistical analysis was carried out using STATISTICA 15.5 software (StatSoft Poland, Cracow). Before carrying out statistical tests, the assumptions of normality of distribution and homogeneity of variance in the compared groups of volunteers were verified. Normality of distribution was assessed using the Shapiro-Wilk test, whereas homogeneity of variance was assessed using the Levene test and the Brown-Forsythe test. Then, tests for the significance of differences between the groups of volunteers were carried out. For the comparison of 2 groups, the non-parametric Mann-Whitney test was used, and the parametric test t-Student - for the independent samples. To analyse the distribution of quality variables in groups determined by variable M insulin sensitivity expressed in mg/kg FFM/min), Pearson's chi-square test for independence was carried out. Correlation and linear regression analyses were carried out for the independent variables and between the independent variables and the dependent variable M using the Spearman's rank correlation coefficient analysis and Pearson's linear correlation r.

Due to the large number of variables and the fact that said number was greater than the number of observations, special techniques of data analysis were used as described below. To detect an array of factors determining tissue insulin sensitivity in the optimal way, complex regression models were constructed: a linear model with regularization (ridge and lasso regression), a supervised principal components model and a random forest model. A randomly selected learning sample and a test sample were used in the analyses. The learning sample was used for the construction of models and the testing sample was used for the assessment thereof. The division of the set into two samples is consistent with the good practice applied in the construction of models and was aimed at increasing the reliability of the quality assessment of the prepared models. The size of the learning sample was established at 80% of the size of the whole sample. Thus, 120 volunteers were assigned to the learning sample and 30 volunteers - to the test sample. Human subject were assigned to the samples in a random manner taking into account the goodness of fit of variable M in both groups. The result of the clamp test, determining tissue insulin sensitivity, was subjected to categorization with the value of 6 mg/kg FFM/min adopted as the cut-off value. The inventors resigned from the division of variable M according to the median and tertiles in the examined population due to the relationship of these values with the examined sample. The value of 6 mg/kg FFM/min was adopted on the basis of earlier observations by the inventors and on the basis of literature. Such cut-off point results in that 60 human subjects (40% of the examined group) were assigned to the group with reduced insulin sensitivity.

Regression models were constructed for all examined variables and for the individual areas of variables (clinical characteristics, biochemical characteristics, gene expression in whole blood, gene expression in tissues, protein concentrations in blood). It was observed that the areas of variables concerning clinical characteristics, biochemical characteristics and gene expression make it possible to predict insulin sensitivity independently of each other. Models were constructed using STATISTICA Data Miner (StatSoft, 2011]) software and R package (packages: impute (Hastie T, Tibshirani T, Narasimhan B, Chu G. impute: impute: Imputation for microarray data. 2014, R package version 1.32.0), glmnet (Friedman J, Hastie T, Tibshirani R. (2010). Regularization Paths for Generalized Linear Models via Coordinate Descent. Journal of Statistical Software, 2010, 33: 1-22. URL http://www.jstatsoft.org/v33/i01) and superpc (Bair E., Tibshirani R. superpc: Supervised principal components. 2012, R package version 1.09. http://CRAN.R-project.org/package=superpc).

The models described can be divided into two groups - models whose purpose is to best predict the value of variable M on the basis of all variables and models that are easier to be interpreted due to reduced number of predicators. The quality of the goodness of fit of the models from the first group (ridge and random forest regression) is slightly better compared to the remaining ones. On the other hand, the lasso model and the 'supervised principal components' model reduce the number of variables and make it possible to find significant diagnostic factors and thus help to understand the examined phenomenon. In particular, the 'supervised principal components' model, apart from indicating essential factors that have an influence on the value of variable M, shows relations between predicators. To develop the insulin sensitivity index, the 'supervised principal components' model was finally chosen, which predicted variable M in the optimal way with simultaneously reduced number of predicators (unlike in the ridge or random forest models which use all examined variables).

In all analyses presented in the examples of the present invention the adopted statistical significance level was $p < 0.05$.

The applied regression models made it possible to develop the insulin sensitivity index determining tissue insulin sensitivity in the optimal way, also as compared with the above-mentioned indices used before. The index developed in accordance with the invention is independent of insulinaemina and glucose fluctuations and makes it possible to obtain the determination with 80% sensitivity and 78% specificity. The diagnostics power is 0.82 and it is greater than the diagnostic power of previously known indices.

It should be added that the developed method for determining tissue insulin sensitivity and the method for identifying insulin resistance according to the invention were analysed in relation to the tissue insulin sensitivity measured by the clamp technique as a continuous variable, i.e. without categorising human subjects with high and low tissue insulin sensitivity, that is, by the correlation analysis method. In that case the index also proved to be better compared to the previously known indices. It should be noted that the examined group consisted of young, generally healthy human subjects; in such circumstances correlations of different indices with the value obtained in the clamp test are usually weaker than in the case of a test performed in groups of human subjects having a significant level of insulin resistance. In this context, it is worth mentioning that the methods for determining tissue insulin sensitivity and indentifying insulin resistance and/or determining a predisposition for disorder connected with insulin resistance, as developed according to the invention, as well as the insulin sensitivity index according to the invention showed a strong, significant correlation with the value of insulin sensitivity obtained in the clamp test ($r = 0.57$, $p<0.0001$), which is stronger than HOMA-IR ($r = -0.39$, $p < 0.003$) and QUICKI ($r = 0.36$, $p<0.002$). The methods for determining tissue insulin sensitivity and insulin resistance, as developed according to the invention, and the insulin sensitivity index according to the invention also proved to be better in relation to the Matsuda index (the correlation Matsuda vs. the value from the clamp technique: $r = 0.38$, $p < 0.001$), which is even more important considering that the Matsuda index requires the measurement of glucose and insulin concentrations in the blood during a 2-hour glucose tolerance test, whereas the developed method for determining tissue insulin sensitivity and the insulin sensitivity index according to the invention requires only a fasting blood sample from one blood draw.

To sum up, according to the present invention, a method for determining tissue insulin sensitivity and a method for identifying insulin resistance based on *in vitro* measurements of the expression of indicated genes have been developed. The methods and kits according to the invention make it possible to determine tissue insulin sensitivity of the examined human subject and to obtain a specific numerical value determining tissue insulin sensitivity, and by relating it to the recommended values, to determine whether insulin sensitivity is reduced or not. This enables a precise identification of insulin resistance, early diagnosis of insulin resistance on the basis of results obtained from a single fasting blood draw.

The above-described insulin sensitivity index according to the invention is suitable for routine application in clinical practice, for tests in general population, in human subjects with normal body weight, in particular in human subjects: with a positive family history of diabetes, with increased fasting glycaemia, with impaired glucose tolerance, overweight, obesity, with lipid disorders, with dyslipidaemia, with metabolic syndrome, with fatty liver, with cardiovascular diseases, including hypertension, with atherosclerosis, with ischaemic heart disease, after cerebral stroke, with neurodegenerative diseases, in women who had gestational diabetes or gave birth to a baby with over 4 kg body weight, or in first-degree relatives of a person suffering from diabetes and/or cardiovascular diseases, or persons before bariatric surgery and/or after such surgery.

According to the invention, any primers for the amplification of the indicated genes can be used, without limitation to the above-indicated preferable pairs of primers; however, the primers must be highly specific to the amplified sequence. In the case of Real Time PCR, the same principles of primer design are used as in the classic PCR. An optimal primer length is between 18 and 22 nucleotides. The difference between the melting temperatures of primers from one pair should not be higher than 5°C. The standard melting temperature should conveniently fall within the range of 52-60°C. The higher the melting temperature the lower the risk of obtaining non-specific reaction products. In order to obtain a high PCR efficiency one should avoid primers forming secondary structures or primer-primer structures between the oligonucleotides of one pair of primers, since in such situation, it is possible to obtain not only a signal from the product of interest but also a signal from the non-specific reaction products or secondary structures formed between primers.

### Brief Description of Drawings

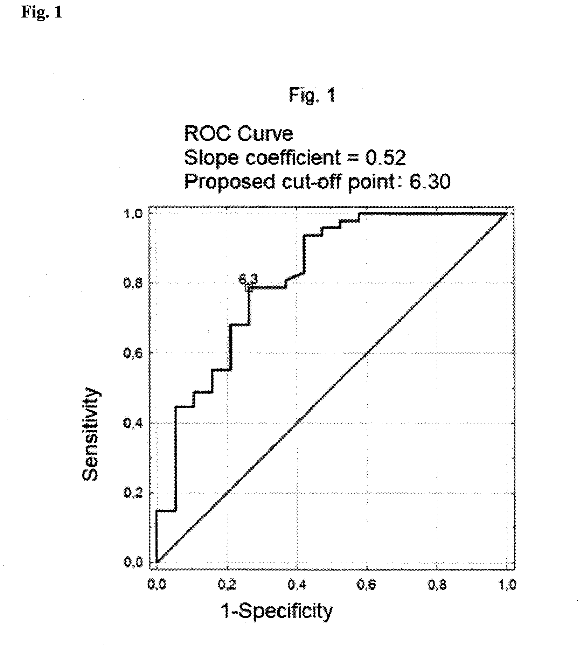

Figure 1 shows a curve for the testing sample. For the cut-off point of 6.30 the sensitivity of the developed insulin sensitivity index according to the invention is 80%, whereas the specificity is 78%.

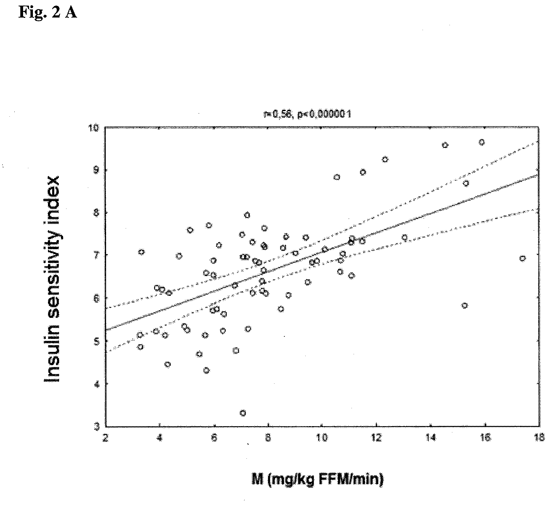

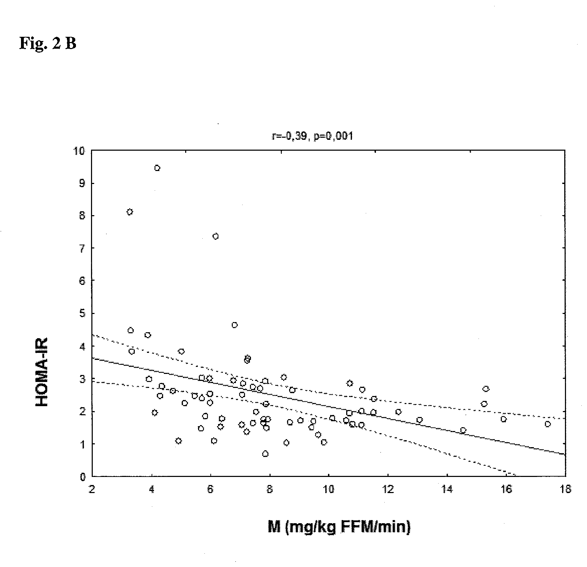

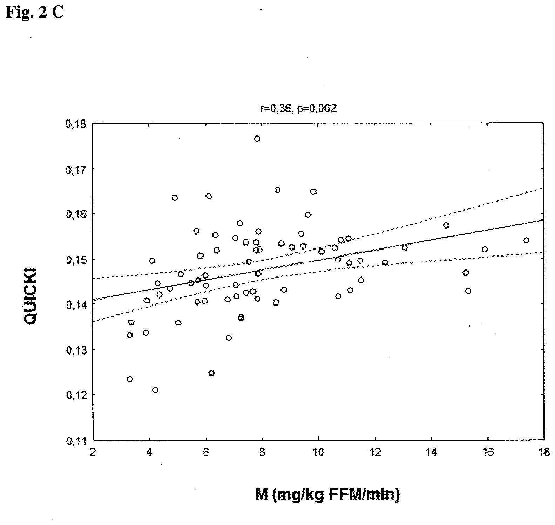

Figure 2 shows the results of the analysis of the correlation between the insulin sensitivity index according to the invention and the results of insulin sensitivity obtained in the clamp test, and with the use of HOMA-IR and QUICKI indices. In Figure 2 A, the statistically significant correlation with the value of variable M ($r=0.57$, $p<0.000001$) can be seen. Figure 2 B shows the obtained statistically significant correlation which is stronger than that using HOMA-IR index ($r=-0.34$, $p=0.00002$), whereas Figure 2 C shows the statistically significant correlation with the QUICKI index ($r=0.34$, $p=0.00002$).

### Examples

*Examined group*

150 generally healthy human subjects aged 18 - 35: 117 men and 33 women were enrolled in the study. Volunteers for the study were recruited through local advertisements and among medical personnel and students. All examined human subjects were non-smokers, without concomitant diseases (ischaemic heart disease, hypertension, infections, allergies, diabetes, neoplasms, diseases of the kidneys, the liver and the endocrine system), without sings of malnutrition and without severe obesity (BMI - body mass index between 18.9 and 40 kg/m$^2$), and were not taking any medicaments. Their body weight was stable in the previous 3 months. None of the women had any menstrual disorders, all of them were examined between the 3$^{rd}$ and 5$^{th}$ day of the menstrual cycle.

The inventors received an approval of the Bioethics Committee of the Medical University in Bia ļystok to carry out the study. All the examined human subjects gave a written consent for their participation in the study after reading information concerning the study and explaining any doubts with a practitioner participating in the project.

*Study protocol and methods*

Stage I

*Medical examination and anthropometric measurements*

The study was carried out in two stages. All tests were carried out under fasting conditions, after at least 10-hour period without meal. In the first stage, full medical examination was performed and anthropometric measurements were made in subsequently examined volunteers. The following measurements were taken: body weight, height, waist circumference (the smallest circumference between the costal arch and the iliac crest), hip circumference (the widest place between the waist and thighs), body fat percentage by bioelectrical impedance analysis method using Tanita TBF-511 Body FAT Analyzer (Tanita Copr., Tokio, Japan). Then, BMI was calculated as body mass x height $^{-2}$ and expressed in kg/m$^2$, Waist-to-Hip Ratio (WHR), body Fat Mass (FM) and Fat-Free Mass (FFM) on the basis of body mass and fat percentage in the organism. In further tests were enrolled human subjects who were non-smokers, without concomitant diseases, who had no deviations in the basic examination and who were not taking any medicaments were eligible.

*Glucose tolerance assessment and preliminary biochemical tests*

After the initial medical qualification, a standard oral glucose tolerance test (OGTT) was performed. During the test the blood was drawn from the basilic vein in fasting conditions, before the administration of glucose, and in 30$^{th}$, 60$^{th}$ and 120$^{th}$ minute after oral 75g-glucose load, in order to determine plasma glucose concentrations as well as insulin and free fatty acids concentrations in the serum. Plasma glucose concentrations were directly determined during the examination. Additionally, the following standard laboratory parameters were directly determined in the fasting blood with the use of standard reagent kits and diagnostic apparatuses: complete blood count with differential, coagulation, lipids, electrolytes, uric acid, creatinine clearance, bilirubin, total protein content and proteinogram, the activity of: aspartate aminotransferase (AspAt), alanine aminotransferase (AlAT), alkaline phosphatase, gamma-glutamyl transpeptidase (GGTP); iron concentration, ferritin concentration, total iron binding capacity TIBC), C-reactive protein (hsCRP), TSH, fT3 and fT4, cortisol and sex hormones. Urinalysis and microalbuminuria test were also performed in a routine way. The samples of serum and plasma were stored at -80°C until the performance of the remaining analyses. Human subjects who were enrolled in the second stage of the study had normal glucose tolerance and normal results of the laboratory tests panel.

Stage II

In stage II, a biopsy of the vastus lateralis muscle and the subcutaneous adipose tissue was performed and then tissue insulin sensitivity was measured using the hyperinsulinemic-euglycemic clamp technique in the known way as described, e.g., in the following publications: Karczewska-Kupczewska M, Lelental N, Adamska A, Niko ļajuk A, Kowalska I, Górska M, Zimmermann R, Kornhuber J, Str ączkowski M, Lewczuk P. The influence of insulin infusion on the metabolism of amyloid β peptides in plasma. Alzheimers Dement 2013, 9: 400-405; and Karczewska-Kupczewska M, Kowalska I, Nikolajuk A, Adamska A, Zielińska M, Kamińska N, Otziomek E, Górska M, Str ączkowski M. Circulating brain-derived neurotrophic factor concentration is downregulated by intralipid/heparin infusion or high-fat meal in young healthy male subjects. Diabetes Care 2012, 35: 358-362. The measurements were performed at least 5 days after OGTT, in the morning, in fasting conditions.

*Biopsy of vastus lateralis muscle and subcutaneous adipose tissue*

Prior to the clamp test, a biopsy of the vastus lateralis muscle and the subcutaneous adipose tissue from the umbilical region was performed under local anaesthetic: 1 % lidocaine, after a small incision of the skin (about 1 cm). The biopsy of the muscle was performer using a special needle (Popper and Sons, New Hyde Park, NY, USA), about 15 cm above the patella (knee cup). The biopsy of the adipose tissue was performed using a Byron Accelerator III needle.

The material from the biopsy of tissues was placed in -80°C in order to preserve it until RNA and protein were isolated. Gene expression in the tissues was measured by an mRNA microarray technique, and then Real Time PCR method.

When carrying out all examinations and test described herein, standard generally known procedures for preparing biological material, biochemical analyses, RNA isolation and gene expression measurement were used, as well as commercially available kits and apparatuses for those purposes, in accordance with the producers' recommendations, unless otherwise indicated.

*Example 1*

*Measurement of tissue insulin sensitivity and identification of high and low tissue insulin sensitivity, as well as measurement of gene expression by mRNA microarray method*

In order to perform the test using the clamp technique, 2 IV needles were inserted into the volunteers' 2 basilic veins. One of the needles was intended for simultaneous infusion of glucose and insulin, whereas the other one, placed on the opposite side, was used for drawing blood in order to determine current glycaemia. The limb from which the blood was drawn was heated by means of an electric cushion up to about 60° C in order to arterialize venous blood. During the test, which lasted 2 hours, intravenous infusion of short-acting insulin (Actrapid HM, Novo Nordisk, Copenhagen, Denmark) was used: in the first 5 min - at the rate of $80\,mU \times m^{-2} \times min^{-1}$, from $5^{th}$ minute - $60\,mU \times m^{-2} \times min^{-1}$, and then, from 10th min continuous infusion at the rate of $40\,mU \times m^{-2} \times min^{-1}$, to obtain stable hyperinsulinemia, sufficient to inhibit endogenous insulin secretion, to suppress lipolysis and reduce endogenous glucose production to 10-15% of the baseline values. Glucose concentration during the clamp test was determined every 5 minutes using YSI 2300 Stat Plus analyser. Minimal amount of blood was needed to determine glycaemia and the result was obtained after 15-45 sec. During the clamp test the analyser was automatically calibrated - at least every 30 min. In the $4^{th}$ minute of the examination, the infusion of 20% glucose (1.11 mol/l) began, initially at the rate of $2\,mg \times kg^{-1} \times min^{-1}$, then from $10^{th}$ minute, glucose infusion was controlled manually depending on the current glucose concentrations, so that glycaemia was closest to 90 mg/dl (5.0 mmol/l). In all human subjects, glucose flow rate was controlled empirically.

When using the above-described protocols, stable metabolic conditions are obtained - stable hyperinsulinemia. In the second hour of the test stable glucose concentrations and stable glucose flow rate are obtained. In such circumstances, glucose flow rate equals the rate of glucose metabolism in tissues, primarily in the skeletal muscles. The adopted value of the insulin-dependent glucose uptake in the organism is the average glucose infusion rate in the last 40 minutes of the clamp test, corrected taking into account slight changes in glycaemia in this time interval and converted into FFM (value M).

The result of the clamp test determining tissue insulin sensitivity was subjected to categorization with the value of 6 mg/kg FFM/min adopted as the cut-off point. The inventors resigned from the division of the quality variable (tissue insulin sensitivity) according to the median and tertiles in the examined population due to the relationship of these values with the examined sample. The value of 6 mg/kg FFM/min was adopted on the basis of earlier observations and on the basis of literature.

Moreover, blood was taken prior to the clamp test in order to measure concentrations of selected proteins in serum, to perform proteomic analysis and isolation of peripheral blood mononuclear cells (PBMC) in order to carry out the analyses of gene expression using the mRNA microarray and Real Time PCR method. An analysis of expression the selected genes in whole blood was also performed using Real Time PCR method.

*Isolation of peripheral blood mononuclear cells (PBMC)*

Isolation of PBMC was performed under baseline conditions (under fasting conditions, prior to the clamp test) and after the clamp test (in $120^{th}$ minute). Blood samples (about 8 ml) were drawn to BD Vacutainer® CPT™ Cell Preparation Tubes (Becton Dickinson AG, NJ, USA) with sodium citrate. The blood was mixed with sodium citrate by turning over the tube 10 times, incubated at room temperature for 20 minutes and then centrifuged, at room temperature with a relative centrifugal force (RCF) of 1800 for 30 minutes. After centrifugation, PBMC and platelets were in a whitish layer just below the plasma layer, which was gently discarded without destroying the PBMC layer. The cells were transferred by means of a Pasteur pipette to a 15 mL Falcon Conical Tube with a threaded closure and phosphate buffered saline (PBS) was poured up to the volume of 15 ml. The tube was centrifuged for 15 minutes with 300 RCF, at room temperature, and then the supernatant was discarded without destroying the cell pellet. The pellet was broken by gently knocking the tube and then PBS (10 mL) was added again. Centrifugation was repeated - 10 minutes, at 300 RCF, at room temperature, and then the supernatant was discarded, PBMC pellet was suspended in 80 $\mu$l of a RNA stabilizing agent (RNALater, Ambion, Thermo Scientific Inc., Wilmington, DE, USA). All procedures described above were carried out within 2 hours from the blood draw. The samples were frozen at -80°C until carrying out further analyses.

*Isolation of RNA*

From muscular tissue

The material collected during biopsy (about 60-80 mg) was removed from a test material stabilising reagent (RNA Later, Ambion) and then it was washed with PBS. The muscular tissue was homogenised using TissueRuptor (Qiagen) on ice for about 40 seconds, in 300 $\mu$l of RLT buffer with the addition of $\beta$-mercaptoethanol. Homogenisation consists in that cell structures are destroyed as a result of rotation of the homogeniser's tip at 35000 rpm. It is recommended to use replaceable autoclaved reusable tips, which prevents contamination of samples. Homogenized samples were placed in an apparatus for automatic isolation (QiaCube, Qiagen). RNA from the homogenate was isolated using RNeasy Fibrous Tissue Kit (Qiagen) using silica resin which binds nucleic acids. Chaotropic salts contained in the kit for isolation cause the breakdown of cell membranes and denaturation of proteins, which promotes isolation of nucleic acids. RNA was suspended in 40 $\mu$l of RNase-free water and it was immediately frozen at -80 °C for further analyses.

From fatty tissue

Fatty tissue (about 100 mg) derived from biopsy was removed from a test material stabilising reagent (Allprotect Tissue reagent, Qiagen) and it was washed with PBS. Then, it was homogenised in 1 ml of a reagent for destroying structures of adipocytes (QIAzol Lysis Reagent, Qiagen) for about 30 seconds. The homogenised samples were placed in an apparatus for automatic isolation (QiaCube, Qiagen). RNA was isolated from fat using a kit dedicated for this type of tissue. (RNeasy Lipid Tissue Mini Kit, Qiagen). RNA was suspended in 30$\mu$l RNase-free water and it was immediately frozen at -80 °C for further analyses.

From peripheral blood mononuclear cells (PBMC)

Peripheral blood mononuclear cells (PMBC) stored in a reagent stabilising material (RNA later, Ambion) were thawed and centrifuged at the speed of 300 g for 5 minutes. The supernatant was removed and 350 $\mu$l homogenisation buffer RLT with the addition of $\beta$-mercaptoethanol was added to the cell pellet. The prepared samples were placed in a device for automatic isolation (QiaCube, Qiagen). RNA was isolated from PBMCs using columns for automatic homogenisation (QIAshredder spin column, Qiagen) and a RNeasy Mini Kit (Qiagen). RNA from cells was suspended in 30$\mu$l RNase-free water and it was immediately frozen at - 80 °C for further analyses.

From whole blood

Whole blood was collected in PAXgene™ tube which contains a reactant stabilising the total intracellular RNA. The tubes with the blood, stored at -80°C, were allowed to heat up at room temperature for minimum 2 hours and then they were centrifuged at the speed of 3000 g for 10 minutes. The supernatant was removed and the pellet was suspended in water and centrifuged again. The obtained pellet was transferred to a clean Eppendorf tube and placed in an apparatus for automatic isolation (QiaCube, Qiagen). RNA from whole blood was isolated using PAX gene Blood RNA kit (Qiagen). RNA from cells was suspended in 60 $\mu$l RNase-free water and it was immediately frozen at -80 °C for further analyses.

*Quantitative and qualitative assessment of RNA*

RNA from all isolation procedures was subjected to a qualitative and quantitative assessment. The amount of RNA was measured using NanoDrop 2000 (ThermoScientific). Nucleic acids absorb ultraviolet (UV) light within the range of between 210 nm and 300 nm, with the maximum at 260 nm. The purity of material was assessed on the basis of the ratio of absorbance (A) at 260 nm and 280 nm (A260/A280). For RNA, the ratio was approximately A260/A280 = 2.0. The assessment also includes the wavelength of 230 which indicates contaminations deriving from carbohydrates, proteins and phenol. The A260/A230 value was about 2.2.

RNA was also qualitatively assessed using Bioanalyzer 2100 (Agilent), which uses a capillary electrophoresis technique. It makes it possible to detect RNA degradation by means of RNA Integrity Number (RIN) index and determines the ratio of the amount of material in subunits 18S and 28S. For further analyses only samples with RIN 9-10 were selected.

Additionally, in order to remove all DNA contaminations, RNA samples were subjected to by DNase digestion. Turbo DNA-free Kit (Ambion) was used for this purpose. The presence of DNA, if any, in the samples was verified using PCR (FastStartTaq DNA Polymerase, dNTPPack, Roche). As a standard 10 pM/$\mu$l $\beta$-globulin primers having the following sequences were used:

F1: 5' GTACGGCTGTCATCACTTAG 3' (sequence identification number 13)

R1: 5'CCTGAGACTTCCACACTGAT 3' (sequence identification number 14).

The product of PCR was place on a chip DNA 7500 Agilent in order to verify DNA product in RNA samples.

*Analysis of mRNA microarrays*

The microarray analysis was carried out in RNA samples isolated from fatty tissue, muscles and PBMC. In PBMC, the analyses were carried out at two points of time - at 0 and 120th minute of the clamp. This method makes it possible to analyse the full gene profile (transcriptome) and it is not burdened with an *a priori* research hypothesis. RNA samples were qualified for the microarray experiment on the basis of RIN. The next stage involved preparing labelled samples of aRNA or cDNA - depending on the selected microarray type to which a given sample was later hybridized. When preparing the labelled material, a number of quality analyses was made. Following the step of amplification, labelling and fragmentation, quality tests were carried out in order to select only those samples that met all quality criteria necessary for hybridization.

The labelled nucleic acid was hybridised to the microarray in a suitably selected temperature depending on the type of microarray. RNA isolated from muscle and fatty tissue, after being labelled, was hybridized to the microarray of the following type: Affymetrix GeneChip Human Genome U133Plus 2.0, and RNA from PBMC - to the GeneChip Human Gene 2.0 st microarray. After the step of hybridisation, washing and 'staining' was performed in washing stations so as to obtain the image of microarray in the next step, using the device: GeneChip Scanner 3000 7G. The Affymetrix Launcher software generated CEL files for each hybridised sample, which were imported to Partek Genomics Suite software and then normalisation, qualitative analysis of the samples and bioinformatic analysis were carried out. Initial assessment of expression profile repeatability was performed using the Principal Component Analysis. To detect genes having significantly altered expression, the analysis of variance (ANOVA) was used. Then, groups of genes and samples having similar expression profiles were determined using the Hierarchical Clustering analysis. The functional analysis of obtained lists of genes was carried out Ingenuity Pathway Analysis software.

*Analytical methods*

Plasma glucose concentration was measured using an enzymatic method with the use of a biochemical analyser (YSI 2300 STAT PLUS). Serum lipids concentration was determined by a calorimetric method with the use of Cobas c111 analyser (Roche Diagnostics, Mannheim, Germany). The activity of: AspAt, AlAt was determined by a kinetic spectrophotometric method with the use of ARCHITECTc4000 analyser (the total coefficient of variation - CV for AlAt was below 5.3 %). Concentration of hsCRP was measured using a nephelometric method (Dade Behring, Marburg, Germany).

Serum insulin concentration was measured using an immunoradiometric method (RMA; DIAsource ImmunoAssays S.A., Nivelles, Belgium) having 1 mIU/ml sensitivity as well as intratest and intertest values CV which were: below 2.2% and 6.5%, respectively. Serum FFA concentration was measured using a commercially available kit (Wako Chemicals, Richmond, VA, USA). The islet cell antibodies titre was also determined.

All parameters were determined using known methods and commercially available kits and analysers in accordance with producers' recommendations.

*Statistical analysis*

The statistical analysis was carried out using STATISTICA 15.5 software (StatSoft Poland, Cracow). Before carrying out statistical tests, the assumptions of normality of distribution and homogeneity of variance in the compared groups of volunteers were verified. Normality of distribution was assessed using the Shapiro-Wilk test, whereas homogeneity of variance was assessed using the Levene test and the Brown-Forsythe test. Then, tests for the significance of differences between the groups of volunteers were carried out. For the comparison of 2 groups, the non-parametric Mann-Whitney test was used, and the parametric test t-Student - for the independent samples. To analyse the distribution of quality variables in groups determined by variable M, Pearson's chi-square test of independence was carried out. Correlation and simple regression analyses were carried out for the independent variables and between the independent variables and the dependent variable M using the Spearman's rank correlation coefficient analysis and Pearson's linear correlation r.

Due to the nature of the problem, i.e. a great number of variables and the fact that it was greater than the number of observations, special data analysis techniques were used. In order to detect an array of factors determining tissue insulin sensitivity in the optimal way, complex regression models were constructed: a linear model with regularization (lasso regression) and a supervised principal components model. The stability paths method was applied which is used to limit the number of variables incorporated in the model but which are not related to the examined phenomenon. This method used the results of lasso models applied to a multiple sampled subset (e.g. half of cases).

A randomly selected learning sample and a testing sample were used in the analyses. The learning sample is used to construct models and the testing sample - for the assessment thereof. The division of a set into two samples is consistent with the good practice used in the construction of models and it is aimed at increasing reliability of the quality assessment of the prepared models. The size of the learning sample was established at 80% of the size of the whole sample. Thus, 120 volunteers were assigned to the learning sample and 30 volunteers - to the testing sample. In the case of gene expression in whole blood the results were available in 73 human subjects due to loss of part of the material. In such circumstances the learning sample numbered 58 human subjects and the testing sample - 15. Human subjects were assigned to the samples in a random manner taking into account the goodness of fit of variable M in both groups.

Regression models were constructed for all examined variables and for the individual areas of variables (clinical characteristics, biochemical characteristics, gene expression in whole blood, in tissues). To develop the index, gene expression in whole blood was used. The models were constructed using STATISTICA Data Miner software (StatSoft, 2011) and R package (packages: impute, glmnet and superpc). In all analyses the adopted statistical significance level was $p < 0.05$

*Results*

It was observed that the variable areas related to the clinical and biochemical characteristics and gene expression in whole blood allow to predict insulin sensitivity independently of each other.

The clinical characteristics of human subjects with high and low tissue insulin sensitivity determined according to the clamp test are presented in Table 2.

Human subjects with low tissue insulin sensitivity were characterized by higher systolic pressure ($p = 0.046$), body weight ($p = 0.002$), BMI ($p = 0.013$), waist circumference ($p = 0.0004$), hip circumference ($p = 0.001$) and Waist-to-Hip Ratio (WHR, $p = 0.039$), plasma glucose concentration ($p = 0.023$), serum insulin concentration ($p = 0.0027$), higher concentrations of total cholesterol ($p = 0.007$), triglycerides ($p = 0.0001$) and LDL cholesterol ($p = 0.037$), and lower HDL cholesterol concentration ($p = 0.03$), higher red blood cells count (RBC, $p=0.0018$), haemoglobin concentration in the blood (Hb, $p = 0.008$), haematocrit (Ht, $p = 0.019$), white blood cells count (WBC, $p = 0.003$), AIAT activity ($p = 0.002$), concentration of sodium ($p=0.001$), uric acid ($p = 0.004$), creatinine clearance ($p = 0.003$), higher TIBC ($p = 0.02$) and ferritin ($p=0.02$) (Table 2 below). In all examined human subjects a negative titre of islet cell antibodies was reported.

Table 2. Clinical characteristics of human subjects with high (M > 6 mg/kg FFM/min; n = 90) and low (M < 6 mg/kg FFM/min; n = 60) tissue insulin sensitivity.

| Parameter | High tissue insulin sensitivity | Low tissue insulin sensitivity |
|---|---|---|
| Age (years) | 23.41±2.76 | 23.37±3.18 |
| Systolic pressure (mmHg) | 121.53±14.63 | 125.80±9.21 * |
| Diastolic pressure (mmHg) | 76.38±8.22 | 78.32±7.69 |
| Body weight (kg) | 76.67±13.87 | 84.48±17.03* |
| BMI (kg/m$^2$) | 24.47±3.67 | 26.14±4.44* |
| Waist circumference (cm) | 84.91±9.70 | 91.65±12.97* |
| Hip circumference (cm) | 98.29±8.90 | 103.10±8.50* |
| WHR | 0.86±0.06 | 0.89±0.08* |
| % fat | 21.72±8.86 | 22.85±9.06 |
| Plasma glucose (mg/dl) | 85.53±7.92 | 88.67±8.64* |
| Serum insulin ($\mu$IU/ml) | 10.10±5.22 | 13.01±6.42* |
| Serum FFA (mmol/l) | 0.65±0.26 | 0.72±0.28 |
| Cholesterol (mg/dl) | 164.77±29.01 | 178.84±32.38* |
| Triglycerides (mg/dl) | 76.48±32.40 | 104.65±54.67* |
| HDL cholesterol (mg/dl) | 61.31±12.34 | 56.98±10.73* |
| LDL cholesterol (mg/dl) | 95.83±28.66 | 107.26±36.71* |
| RBC (mln/$\mu$l) | 4.96±0.42 | 5.17±0.34* |
| Hb (g/dl) | 14.64±1.21 | 15.13±0.90* |
| Ht (%) | 43.32±3.42 | 44.53±2.46* |
| WBC (thousand/$\mu$l) | 5.90±1.11 | 6.50±1.33* |
| Blood platelets (thousand/$\mu$l) | 240.18±41.63 | 244.17±48.62 |
| Fibrinogen (mg/dl) | 281.82±49.60 | 287.22±51.07 |
| AspAt (U/l) | 23.70±9.56 | 23.92±7.47 |

(continued)

| Parameter | High tissue insulin sensitivity | Low tissue insulin sensitivity |
|---|---|---|
| AlAT (U/l) | 21.98±15.64 | 28.58±13.12* |
| Sodium (mmol/l) | 138.53±2.36 | 139.87±2.57* |
| Potassium (mmol/l) | 4.36±0.31 | 4.42±0.35 |
| Total calcium (mmol/l) | 2.43±0.12 | 2.46±0.11 |
| Ferrum ($\mu$g/dl) | 106.41±44.37 | 94.02±41.48 |
| TIBC ($\mu$g/dl) | 304.67±93.65 | 338.84±74.28* |
| Ferritin (ng/ml) | 60.28±47.16 | 78.66±47.99* |
| Uric acid (mg/dl) | 4.80±1.12 | 5.34±.13* |
| Creatinine (mg/dl) | 0.92±0.19 | 0.93±0.11 |
| Creatinine clearance (ml/min) | 131.82±26.40 | 146.16±31.11* |
| Total protein (g/dl) | 7.37±0.39 | 7.47±0.35 |
| Albumins (%) | 61.21±3.66 | 62.23±4.04 |
| $\beta$-globulins (%) | 11.68±1.20 | 12.09±1.46 |
| $\gamma$-globulins (%) | 15.61±8.02 | 13.85±2.49 |
| hsCRP (mg/l) | 0.61±0.56 | 0.81±0.77 |
| M (mg/kg FFM/min) | 8.94±2.51 | 4.57±1.04* |

* $p < 0.05$ vs. group with high insulin sensitivity

*Example 2*

*Gene expression measurement using a reverse transcription and Real Time PCR method.*

The expression of genes selected based on the analyses of PBMC microarrays was subsequently verified using Real Time PCR in whole blood.

*Reverse transcription*

Verified RNA was transcribed to cDNA using QuantiTect Reverse Transcription, Qiagen kit. 0.5 $\mu$g total RNA was used for the reaction. The reaction proceeds in 2 steps. In the first step, genomic DNA is eliminated. This involves purifying RNA samples by short incubation in gDNA Wipeout Buffer at 42°C for 4 min. In the second step, a reverse transcription takes place. The following are added: Quantiscript Reverse Transcriptase (obtained from *E.coli*), Quantiscript RT Buffer 5x (dNTP) and RT Primer Mix (a mixture of oligo-dT and Random primer, which ensures high-quality cDNA comprising all RNA transcript regions, even from 5' region). The sample so prepared was incubated at 42°C for 15 min, and then at 95°C for 3 min when reverse transcriptase is deactivated.

*Analysis of tissue gene expression using Real Time PCR*

Tissue gene expression assessed by the microarray analysis which to the greatest extent differentiated groups of human subjects with high and low tissue insulin sensitivity and which changed the most under the influence of insulin was subsequently verified in the real time polymerase chain reaction (Real Time PCR). PBMC gene expression obtained in the microarray analysis which to the greatest extent differentiated groups of human subjects with high and low tissue insulin sensitivity and which changed the most under the influence of insulin was confirmed in whole blood by the Real Time PCR. Moreover, after an initial statistical analysis, the expression of genes selected on the basis of tissue microarrays analysis was assessed using the method of Real Time PCR in whole blood.

The expression of the selected genes was examined by the Real Time PCR method with the use of specific primers and TaqMan Real Time PCR probes, using Light Cycler 480II Roche device (Roche Diagnostics GmbH, Mannheim, Germany). The following composition of the reaction mixture was used in the reaction: 5 $\mu$l LightCycler 480 Probes Master (reaction mixture comprising FastStart Taq DNA polymerase), 0.5 $\mu$l Real-time Ready Assay - a mixture of TaqMan primers and probes (the final concentration of primers: 8 pmol and TaqMan probe: 4 pmol), 1 $\mu$l of obtained cDNA (the final concentration: 75 pg). The whole mixture was supplemented with water (PCR Grade) to the final volume of 10 $\mu$l.

PCR reaction was performed in the following reaction conditions:

- the step of initial denaturation and DNA polymerase activation (10 min, 95°C),
- 45 cycles of amplifying the tested DNA performed in 3 steps: denaturation (10 s, 95°C), primer annealing (30 s, 60°C) and synthesis (72°C, 1 s),
- cooling the device (30 s, 40°C).

RNA samples were tested for the presence of residual DNA using a polymerase chain reaction (PCR) with the use of primers complementary to β-globulin gene having the concentration of 10 pM/μl (F1: 5'GTACGGCTGTCATCACT TAG 3'; R1:5'CCTGAGACTTCCACACTGAT 3'; sequence identification numbers 13 and 14, respectively).

The PCR reaction was performed in the following way: initial denaturation (4 min., 94°C), 35 cycles of: denaturation (45 s., 95°C), primer annealing (45 s., 57°C) and synthesis (60 s., 72°C)). 2.5 μl of purified RNA, as described above, was used for the reaction.

The tests were performed in triplicate. Real-Time Ready Custom RT-qPCR (Roche Diagnostics GmbH) assays were used. Glyceraldehyde-3-phosphate dehydrogenase (GAPDH) was used as a reference gene. Calculations were performed using Light Cycler 480 Software 1.5.0 SP3. It compares the level of two different target sequences in a single sample (the tested gene and the reference gene), and the final result is expressed as the ratio of those genes' levels. The ratio of the target gene to the reference gene is a relative, non-dimensional number; it is important only for the comparison between samples. Primer sequences used are presented in Table 3.

Table 3. Primer sequences

| Gene | Id. No. in the test | Sequence Id. No. in the Sequence listing | Forward primer | Reverse primer | Sequence Id. No. in the Sequence listing |
|---|---|---|---|---|---|
| *KLF9* | 126368 | 1 | 5'-CTCCGAAAAGAGGCACAAGT | 5'-CGGGAGAACTTTTTAAGGCAGT | 2 |
| *PEMT* | 127584 | 3 | 5'-GGCCTCGGCAATATTGATT | 5'-GTCCACGTAGCCCAGCAG | 4 |
| *EGR1* | 100904 | 5 | 5'-AGCACCTGACCGCAGAGT | 5'-GGCAGTCGAGTGGTTTGG | 6 |
| *GAPDH* | 101128 | 7 | 5'- CTCTGCTCCTCCTGTTCGAC | 5'-ACGACCAAATCCGTTGACTC | 8 |

Template sequences (cDNA), sequences of respective amplicons of the above-indicated genes, for the above-indicated primers are given in the attached Sequence listing.

Sequence for KLF9 gene:

CTCCGAAAAGAGGCACAAGTGCCCCTACAGTGGCTGTGGGAAAGTCT ATGGAAAATCCTCCCATCTCAAAGCCCATTACAGAGTGCATACAGGTG AACGGCCCTTTCCCTGCACGTGGCCAGACTGC CTTAAAAAGTTCTCCCG (Sequence Id. No. 9).

Sequence for PEMT gene:

GGCCTCGGCAATATTGATTTTAGACAGGCAGACTTCTGCGTTATGACC CGGCTGCTGGGCTACGTGGAC (Sequence Id. No. 10)

Sequence for EGFR1 gene:

AGCACCTGACCGCAGAGTCTTTTCCTGACATCTCTCTGAACAACGAGA AGGTGCTGGTGGAG ACCAGTTACCCCAGCCAAACCACTCGACTGCC (Sequence Id. No. 11).

Sequence for GAPDH reference gene:

CACTAGGCGCTCACTGTTCTCTCCCTCCGCGCAGCCGAGCCACATCGC TCAGACACCATGGGGAAGGTGAAGGTCGGAGTCAACGGATTTGGTCG TATTGGGC (Sequence Id. No. 12).

Considering the simplicity of a potential index, the analyses were carried out in fasting whole blood. As mentioned previously, apart from the genes selected on the basis of the PBMC analyses, the inventors also took into account the expression of genes in whole blood, the tissue expression of which was to the greatest extent connected with insulin sensitivity in complex regression models: *KLF9* (originally selected in a muscle) and *PEMT* (originally selected in fat). The most significant differences in the expression of the examined genes between groups of human subjects with high and low tissue insulin sensitivity are presented in Table 4, and the results of one-way correlation analysis of the tested parameters (i.e. gene expression in whole blood) with the dependent variable M - in Table 5.

Table 4. Significant differences in gene expression in whole blood between groups with high and low tissue insulin sensitivity.

|  | High insulin sensitivity | Low insulin sensitivity |
|---|---|---|
| Log2 *KLF9* | -4.94±0.53 | -4.65±0.39* |
| Log2 *PEMT* | -6.80±0.64 | -7.17±0.51* |
| Log2 *EGR1* | -6.71±0.85 | -7.16±0.72* |

Table 5. Correlations of the most significant parameters examined in fasting conditions with variable M

|  | Pearson's R | p |
|---|---|---|
| Log2 *KLF9* | -0.41 | 0.0004 |
| Log2 *PEMT* | 0.37 | 0.002 |
| Log2 *EGR1* | 0.37 | 0.002 |

Then, the above-described complex regression models were applied. On that basis the tissue insulin sensitivity index according to the present invention has been developed and its parameters are included in Table 6 below.

Table 6. Coefficients of the model from the area of gene expression in whole blood that predict variable M in the best way.

| Variable | Mean | S.D. | Coefficient |
|---|---|---|---|
| Free text | | | 7.2109 |
| Log2 KLF9 | -4.86 | 0.51 | -1.8717 |
| Log2 PEMT | -6.90 | 0.63 | 1.2903 |
| Log2 EGR1 | -6.84 | 0.84 | 0.1145 |

S.D. - standard deviation.
Coefficient - coefficients of an equation that makes it possible to determine the predicted value of variable M on the basis of original (non-standardized) predicator values.

Measures of the quality of the goodness-of-fit of the model are presented in Table 7.

Table 7. Measures of the quality of the goodness-of-fit for the presented model.

| Model | Characteristics |
|---|---|
| N variables | 34 |
| N model | 3 |
| $R^2$ | 0.31 |
| R | 0.56 |
| RMSE | 1.91 |
| AUC | 0.82 |

N variables - the number of independent variables in the data set used in the construction of the model,
N model - the number of independent variables used in the model,
$R^2$ - coefficient of determination,
R - Pearson coefficient of correlation (Pearson's R) between the observed and predicted values of dependent variable M,
RMSE- root-mean-square error
AUC - AUC value (the area under ROC curve)

The area under the Receiver Operating Characteristic (ROC) curve for the test sample demonstrates that the obtained diagnostic power was 0.82.
The sensitivity and specificity graphs illustrate how the sensitivity and specificity of the test changes depending on the cut-off point. Figure 1 shows a curve for the testing sample. For the cut-off point of 6.30, the sensitivity of the developed insulin sensitivity index is 80%, whereas the specificity is 78%.

*Example 3*

*A comparison of the insulin sensitivity index according to the invention with other methods and indices of insulin sensitivity/insulin resistance.*

Differences between the developed method for determining insulin sensitivity and insulin sensitivity index according to the invention and other methods and indices of insulin sensitivity/insulin resistance known in this field were also studied. A group of human subjects with low insulin sensitivity showed a higher HOMA-IR index (p = 0.003) and lower QUICKI index (p = 0.002). The highest statistical significance was observed for the insulin sensitivity index developed by the present inventors - it was significantly lower in the group of human subjects with low insulin sensitivity assessed in the clamp test (p = 0.0005) (Table 8).

Table 8. The developed index and other indices of insulin sensitivity/insulin resistance in the examined groups of human subjects.

|  | High insulin sensitivity | Low insulin sensitivity | p |
|---|---|---|---|
| Insulin sensitivity index | 6.92±1.17 | 5.84±1.03 | 0.0005 |
| HOMA-IR | 2.18±1.06 | 3.35±2.07 | 0.003 |
| QUICKI | 0.15±0.01 | 0.14±0.01 | 0.002 |
| M (mg/kg FFM/min) | 9.34±2.77 | 4.73±0.93 | 0.000000 |

A correlation of the index developed by the present inventors with the insulin sensitivity index result obtained in the clamp test was also analysed and a strong, significant correlation with variable M was demonstrated (r = 0.56, p < 0.000001, Figure 2A), which is stronger than that in the case of HOMA-IR index (r=-0.39, p = 0.001, Figure 2B) and QUICKI index (r = 0.36, p = 0.002, Figure 2C). This means that the presented index reflects tissue insulin sensitivity in a better and more precise way.

*Example 4*

*Identifying insulin resistance and determining a predisposition for a disorder connected with insulin resistance*

The method for identifying insulin resistance and/or determining a predisposition for a disorder connected with insulin resistance was carried out in a volunteer (No. 136) in the way as described above. All tests and analyses were carried out in the way as described above. The following measured values were obtained: *KLF9*: 0.064 (log2 = -3.977), *PEMT*: 0.006 (log2 = -7.345), *EGR1*: 0.013 (log2 = -6.31). Then, the insulin sensitivity index according to the invention was calculated in the following way:

Insulin sensitivity index = 7.2109 - (-3.977) x 1.8717 + (-7.345) x 1.2903 + (-6.31) x 0.1145 = 4.458.

The insulin sensitivity index value of 4.458 was obtained, which was considerably below the cut-off point of 6.3, and the existence of insulin resistance and a predisposition for a disorder connected with insulin resistance was diagnosed in that human subject.

This result was then confirmed using the clamp test in the way and with the use of kits and analysers as described above. M value of 4.289 mg/kg FFM/min was obtained which proves the existence of insulin resistance in that human subject.

Thus, the above results prove that the invention makes it possible to determine the existence of insulin resistance and a predisposition for disorders connected therewith on the basis of the results of measurements obtained from one blood draw in a sensitive, specific, quick and reliable way.

*Example 5*

*Identifying the lack of insulin resistance and determining the lack of a predisposition for a disorder connected with insulin resistance*

The method for identifying insulin resistance and determining a predisposition for a disorder connected with insulin resistance was carried out in a volunteer (No. 125) in the way as described above. All tests and analyses were carried out in the way and with the use of kits and analysers as described above. The following values of measurements were obtained: *KLF9*: 0.019 (log2 = -5.695), *PEMT*: 0.011 (log2 = -6.493), *EGR1*: 0.036 (log2 = -4.78). Then, the insulin sensitivity index according to the invention was calculated in the following way:

Insulin sensitivity index = 7.2109 - (-5.695) x 1.8717 + (-6.493) x 1.2903 + (-4.78) x 0.1145 = 8.947.

The insulin sensitivity index value of 8.947 was obtained, which was considerably above the cut-off point: 6.3, and thus the existence of high tissue insulin sensitivity and the lack of insulin resistance was identified in that human subject.

This result was then confirmed using the clamp test in the way as described above. M value of 11.518 mg/kg FFM/min was obtained, which proves the lack of insulin resistance in that human subject.

Thus, the above results prove that the invention makes it possible to determine tissue insulin sensitivity, to determine the existence of insulin resistance and a predisposition for disorders connected therewith on the basis of the results of measurements obtained from one blood draw, in a sensitive, specific, quick and reliable way.

**Claims**

1. A method for determining tissue insulin sensitivity in a human subject, **characterized in that**:

   a) RNA is isolated from a whole blood sample drawn from a human subject and the expression of the following genes is measured: KLF9, PEMT and EGR1;
   b) an insulin sensitivity index is determined on the basis of obtained results of the measurements specified in a) using the following equation:

   Insulin sensitivity index = 7.2109 - log2 KLF9 $\times$ 1.8717 + log2 PEMT $\times$ 1.2903 + log2 EGR1 $\times$ 0.1145,

   wherein

   KLF9 is the value of the measured expression of KLF9 gene,
   PEMT is the value of the measured expression of PEMT gene,
   EGR1 is the value of measured expression of EGR1 gene,

   wherein the obtained index value of 6.3 or less indicates a reduced tissue insulin sensitivity,
   and wherein lower result corresponds to lower tissue insulin sensitivity and a higher result corresponds to higher tissue insulin sensitivity (also within the range below and above the cut-off point).

2. A method for identifying insulin resistance in a human subject, **characterized in that**

   a) RNA is isolated from a whole blood sample drawn from a human subject and the expression of the following genes is measured: KLF9, PEMT and EGR1;
   b) an insulin sensitivity index is determined on the basis of obtained results of the measurements specified in a) using the following equation:

   Insulin sensitivity index = 7.2109 - log2 KLF9 $\times$ 1.8717 + log2 PEMT $\times$ 1.2903 + log2 EGR1 $\times$ 0.1145,

   wherein

   KLF9 is the value of measured expression of KLF9 gene,
   PEMT is the value of measured expression of PEMT gene,
   EGR1 is the value of measured expression of EGR1 gene;

   c) insulin resistance is identified when the obtained value of insulin sensitivity index is 6.3 or less.

3. The method according to any one of claims 1 or 2, **characterized in that** in step a) RNA is isolated and the expression of the indicated genes is measured using a reverse transcription method and Real Time PCR method.

4. Use of an insulin sensitivity index defined by the following equation:

   Insulin sensitivity index = 7.2109 - log2 KLF9 $\times$ 1.8717 + log2 PEMT $\times$ 1.2903 + log2 EGR1 $\times$ 0.1145,

   wherein

   KLF9 is the value of measured expression of KLF9 gene,
   PEMT is the value of measured expression of PEMT gene,
   EGR1 is the value of measured expression of EGR1 gene;
   for monitoring tissue insulin sensitivity, according to any one of claims 1 to 3 in particular in type 2 diabetes,
   wherein the obtained index value of 6.3 or less indicates a reduced tissue insulin sensitivity.

5. The use of an insulin sensitivity index according to claim 4, **characterized in that** expression of mRNA of the indicated genes is measured using a reverse transcription method and Real Time PCR method.

6. The method according to claim 3, or use of the index according to claim 5, **characterized in that** in the Real Time PCR method the following primers are used: for KLF9 gene - a forward primer having a sequence No. 1 (5'-CTCCGAA AAGAGGCACAAGT) and a reverse primer having a sequence No. 2 (5'-CGGGAGAACTTTTTAAGGCAGT), for PEMT gene - a forward primer having a sequence No. 3 (5'-GGCCTCGGCAATATTGATT) and a reverse primer having a sequence No. 4 (5'-GTCCACGTAGCCCAGCAG), for EGR1 gene - a forward primer having a sequence No. 5 (5'-AGCACCTGACCGCAGAGT) and a reverse primer having a sequence No. 6 (5'-GGCAGTCGAGTGGTTTGG).

7. The method according to any one of claims 3 or 6, or use of the index according to claim 5 or 6, **characterized in that** mRNA expression of a reference gene, in particular GAPDH, is also measured.

8. The method, or use of the index according to claim claim 7, **characterized in that** in the Real Time PCR method the following primers are used for the reference gene GAPDH: a forward primer having a sequence No. 7 (5'- CTCT GCTCCTCCTGTTCGAC) and a reverse primer having a sequence No. 8 (5'-ACGACCAAATCCGTTGACTC).

**Patentansprüche**

1. Verfahren zur Bestimmung der Gewebeinsulinempfindlichkeit bei einem menschlichen Probanden, **dadurch gekennzeichnet, dass:**

a) RNA aus einer Vollblutprobe eines menschlichen Probanden isoliert und die Expression der folgenden Gene: KLF9, PEMT und EGR1 gemessen wird;
b) ein Insulinsensitivitätsindex auf der Grundlage der erhaltenen Ergebnisse der in a) angegebenen Messungen unter Verwendung der folgenden Gleichung:

Insulinsensitivitätsindex = 7,2109 - log2 KLF9 x 1,8717 + log2 PEMT x 1,2903 + log2 EGR1 $\times$ 0,1145,

bestimmt wird,
wobei

KLF9 der Wert der gemessenen Expression des KLF9-Gens ist,
PEMT der Wert der gemessenen Expression des PEMT-Gens ist,
EGR1 der Wert der gemessenen Expression des EGR1-Gens ist,
wobei der erhaltene Indexwert von 6,3 oder weniger eine verringerte Gewebeinsulinsensitivität anzeigt, und wobei ein niedrigeres Ergebnis einer niedrigeren Gewebeinsulinsensitivität und ein höheres Ergebnis einer höheren Gewebeinsulinsensitivität entspricht (auch innerhalb des Bereichs unterhalb und oberhalb des Grenzwerts).

2. Verfahren zur Erkennung einer Insulinresistenz bei einem menschlichen Probanden, **dadurch gekennzeichnet, dass:**

a) RNA aus einer Vollblutprobe eines menschlichen Probanden isoliert und die Expression der folgenden Gene: KLF9, PEMT und EGR1 gemessen wird;
b) ein Insulinsensitivitätsindex auf der Grundlage der erhaltenen Ergebnisse der in a) angegebenen Messungen unter Verwendung der folgenden Gleichung:

Insulinsensitivitätsindex = 7,2109 - log2 KLF9 x 1,8717 + log2 PEMT x 1,2903 + log2 EGR1 $\times$ 0,1145,

bestimmt wird,
wobei

KLF9 der Wert der gemessenen Expression des KLF9-Gens ist,
PEMT der Wert der gemessenen Expression des PEMT-Gens ist,
EGR1 der Wert der gemessenen Expression des EGR1-Gens ist;

c) Insulinresistenz, wenn der erhaltene Wert des Insulinsensitivitätsindex 6,3 oder weniger beträgt, erkannt wird.

3. Verfahren nach einem der Ansprüche 1 oder 2, **dadurch gekennzeichnet, dass** in Schritt a) RNA isoliert und die Expression der angegebenen Gene unter Verwendung einer Reverse-Transkriptionsmethode und einer Real-Time-PCR-Methode gemessen wird.

4. Verwendung eines Insulinsensitivitätsindex, der durch die folgende Gleichung:

Insulinsensitivitätsindex = 7,2109 - log2 KLF9 x 1,8717 + log2 PEMT x 1,2903 + log2 EGR1 $\times$ 0,1145,

definiert ist,
wobei

KLF9 der Wert der gemessenen Expression des KLF9-Gens ist,
PEMT der Wert der gemessenen Expression des PEMT-Gens ist,
EGR1 der Wert der gemessenen Expression des EGR1-Gens ist;
zur Überwachung der Gewebeinsulinsensitivität nach einem der Ansprüche 1 bis 3, insbesondere bei Typ-2-Diabetes,
wobei der erhaltene Indexwert von 6,3 oder weniger eine verringerte Gewebeinsulinsensitivität anzeigt.

5. Verwendung eines Insulinsensitivitätsindex nach Anspruch 4, **dadurch gekennzeichnet, dass** die Expression von mRNA der angegebenen Gene mittels Reverse-Transkription-Methode und Real-Time-PCR-Methode gemessen wird.

6. Verfahren nach Anspruch 3 oder Verwendung des Index nach Anspruch 5, **dadurch gekennzeichnet, dass** bei der Real Time PCR-Methode folgende Primer: für das KLF9-Gen - ein Vorwärtsprimer mit der Sequenz Nr. 1 (5'-CTC CGAAAAGAGGCACAAGT) und ein Rückwärtsprimer mit der Sequenz Nr. 2 (5'-CGGGAGAACTTTTTAAGGCAGT), für das PEMT-Gen - ein Vorwärtsprimer mit der Sequenz Nr. 3 (5'-GGCCTCGGCAATATTGATT) und ein Rückwärtsprimer mit der Sequenz Nr. 4 (5'-GTCCACGTAGCCCAGCAG), für das EGR1-Gen - ein Vorwärtsprimer mit der Sequenz Nr. 5 (5'-AGCACCTGACCGCAGAGT) und ein Rückwärtsprimer mit der Sequenz Nr. 6 (5'-GGCAGTCG AGTGGTTTGG), verwendet werden.

7. Verfahren nach einem der Ansprüche 3 oder 6 oder Verwendung des Index nach Anspruch 4 oder 5, **dadurch gekennzeichnet, dass** zusätzlich die mRNA-Expression eines Referenzgens, insbesondere GAPDH, gemessen wird.

8. Verfahren oder Verwendung des Index nach Anspruch 7, **dadurch gekennzeichnet, dass** im Real Time PCR-Verfahren für das Referenzgen GAPDH folgende Primer: ein Vorwärtsprimer mit der Sequenznummer 7 (5'-CTC TGCTCCTCCTGTTCGAC) und ein Rückwärtsprimer mit der Sequenznummer 8 (5'-ACGACCAAATCCGTTGAC TC), verwendet werden.

**Revendications**

1. Procédé de détermination de la sensibilité tissulaire à l'insuline chez un sujet humain, **caractérisé en ce que** :

a) l'ARN est isolé à partir d'un échantillon de sang total prélevé sur un sujet humain et l'expression des gènes suivants est mesurée : KLF9, PEMT et EGR1 ;
b) un indice de sensibilité à l'insuline est déterminé sur la base des résultats obtenus des mesures spécifiées au point a) en utilisant l'équation suivante :

Indice de sensibilité à l'insuline = 7,2109 - log2 KLF9 x 1,8717 + log2 PEMT x 1,2903 + log2 EGR1 $\times$ 0,1145,

dans laquelle

KLF9 est la valeur de l'expression mesurée du gène KLF9,
PEMT est la valeur de l'expression mesurée du gène PEMT,
EGR1 est la valeur de l'expression mesurée du gène EGR1,
où la valeur d'indice obtenue de 6,3 ou moins indique une sensibilité tissulaire réduite à l'insuline,
et où un résultat inférieur correspond à une sensibilité tissulaire à l'insuline inférieure et un résultat supérieur correspond à une sensibilité tissulaire à l'insuline supérieure (également dans la plage inférieure et supérieure au point de coupure).

2. Procédé d'identification de la résistance à l'insuline chez un sujet humain, **caractérisé en ce que :**

a) l'ARN est isolé d'un échantillon de sang total prélevé sur un sujet humain et l'expression des gènes suivants est mesurée : KLF9, PEMT et EGR1 ;
b) un indice de sensibilité à l'insuline est déterminé sur la base des résultats obtenus des mesures spécifiées au point a) en utilisant l'équation suivante :

Indice de sensibilité à l'insuline = 7,2109 - log2 KLF9 x 1,8717 + log2 PEMT x 1,2903 + log2 EGR1 x 0,1145, dans laquelle

KLF9 est la valeur de l'expression mesurée du gène KLF9,
PEMT est la valeur de l'expression mesurée du gène PEMT,
EGR1 est la valeur de l'expression mesurée du gène EGR1 ;

c) la résistance à l'insuline est identifiée lorsque la valeur obtenue de l'indice de sensibilité à l'insuline est de 6,3 ou moins.

3. Procédé selon l'une quelconque des revendications 1 ou 2, **caractérisé en ce que** dans l'étape a) l'ARN est isolé et l'expression des gènes indiqués est mesurée en utilisant une méthode de transcription inverse et une méthode de PCR en temps réel.

4. Utilisation d'un indice de sensibilité à l'insuline défini par l'équation suivante :

Indice de sensibilité à l'insuline = 7,2109 - log2 KLF9 x 1,8717 + log2 PEMT x 1,2903 + log2 EGR1 x 0,1145, dans laquelle

KLF9 est la valeur de l'expression mesurée du gène KLF9,
PEMT est la valeur de l'expression mesurée du gène PEMT,
EGR1 est la valeur de l'expression mesurée du gène EGR1 ;
pour surveiller la sensibilité tissulaire à l'insuline selon l'une quelconque des revendications 1 à 3, en particulier dans le diabète de type 2,
dans laquelle la valeur d'indice obtenue de 6,3 ou moins indique une sensibilité tissulaire réduite à l'insuline.

5. Utilisation d'un indice de sensibilité à l'insuline selon la revendication 4, **caractérisée en ce que** l'expression de l'ARNm des gènes indiqués est mesurée à l'aide d'une méthode de transcription inverse et d'une méthode de PCR en temps réel.

6. Procédé selon la revendication 3 ou utilisation de l'indice selon la revendication 5, **caractérisé en ce que** dans le procédé de PCR en temps réel, les amorces suivantes sont utilisées : pour le gène KLF9 - une amorce directe ayant une séquence n° 1 (5'-CTCCGAAAAGAGGCACAAGT) et une amorce inverse ayant une séquence n° 2 (5'-CGG GAGAACTTTTTAAGGCAGT), pour le gène PEMT - une amorce directe ayant une séquence n° 3 (5'-GGCCTCG GCAATATTGATT) et une amorce inverse ayant une séquence n° 4 (5'-GTCCACGTAGCCCAGCAG), pour le gène EGR1 - une amorce directe ayant une séquence n° 5 (5'-AGCACCTGACCGCAGAGT) et une amorce inverse ayant une séquence n° 6 (5'-GGCAGTCGAGTGGTTTGG).

7. Procédé selon l'une quelconque des revendications 3 ou 6 ou utilisation de l'index selon la revendication 4 ou 5, **caractérisé en ce que** l'expression d'ARNm d'un gène de référence, en particulier GAPDH, est également mesurée.

8. Procédé ou utilisation de l'index selon la revendication 7, **caractérisé en ce que** dans le procédé de PCR en temps réel, les amorces suivantes sont utilisées pour le gène de référence GAPDH : une amorce directe ayant une séquence n° 7 (5'-CTCTGCTCCTCCTGTTCGAC) et une amorce inverse ayant une séquence n° 8 (5'-ACGACCAAATCCG TTGACTC).

**Fig. 1**

Fig. 1

ROC Curve
Slope coefficient = 0.52
Proposed cut-off point: 6.30

**Fig. 2 A**

Fig. 2 B

M (mg/kg FFM/min)

**Fig. 2 C**

r=0,36, p=0,002

## REFERENCES CITED IN THE DESCRIPTION

### Non-patent literature cited in the description

- **REAVEN GM**. Role of insulin resistance in human disease. *Diabetes*, 1988, vol. 37, 1595-1607
- **FERNANDEZ-REAL JM** ; **RICART W.** Insulin resistance and chronic cardiovascular inflammatory syndrome. *Endocr Rev*, 2003, vol. 24, 278-301
- **NIGRO J** ; **OSMAN N** ; **DART AM** ; **LITTLE PJ**. Insulin resistance and atherosclerosis.. *Endocr Rev*, 2006, vol. 27, 242-259
- **RORIZ-FILHO J** ; **SA-RORIZ TM** ; **ROSSET I** ; **CAMOZZATO AL** ; **SANTOS AC** ; **CHAVES ML** ; **MORIGUTI JC** ; **RORIZ-CRUZ M.** Pre)diabetes, brain aging, and cognition. *Biochim Biophys Acta*, 2009, vol. 1792, 432-443
- **DEFRONZO RA** ; **TOBIN JD** ; **ANDRES R.** Glucose clamp technique: a method for quantifying insulin secretion and resistance. *Am J Physiol*, 1979, vol. 237, E214-E223
- Assessing insulin sensitivity and resistance in humans. **MUNIYAPPA R** ; **MADAN R** ; **QUON MJ**. Endotext. MDText.com, Inc., 2000
- **ANTUNA-PUENTE B** ; **DISSE E** ; **RABASA-LHORET R** ; **LAVILLE M** ; **CAPEAU J** ; **BASTARD JP**. How can we measure insulin sensitivity/resistance?. *Diabetes Metab*, 2011, vol. 37, 179-188
- **WALLACE TM** ; **MATTHEWS DR.** The assessment of insulin resistance in man. *Diabet Med*, 2002, vol. 19, 527-534
- **FERRANNINI E** ; **MARI A.** How to measure insulin sensitivity. *J Hypertens*, 1998, vol. 16, 895-906
- **MATSUDA M.** Measuring and estimating insulin resistance in clinical research setting. *Nutr Metab Cardiovasc Dis*, 2010, vol. 20, 79-86
- **KO GT** ; **CHAN JC** ; **WOO J** ; **LAU E** ; **YEUNG VT** ; **CHOW CC** ; **COCKRAM CS**. The reproducibility and usefulness of the oral glucose tolerance test in screening for diabetes and other cardiovascular risk factors. *Ann Clin Biochem*, 1998, vol. 35, 62-67
- **LIBMAN IM** ; **BARINAS-MITCHELL E** ; **BARTUCCI A** ; **ROBERTSON R** ; **ARSLANIAN S.** Reproducibility of the Oral Glucose Tolerance Test in Overweight Children. *J Clin Endocrinol Metab*, 2008, vol. 93, 4231-4237
- **RUIGE JB** ; **MERTENS IL** ; **BARTHOLOMEEUSEN E** ; **DIRINCK E** ; **FERRANNINI E** ; **VAN GAAL LF.** Fasting-based estimates of insulin sensitivity in overweight and obesity: a critical appraisal.. *Obesity*, 2006, vol. 14, 1250-1256
- **MALITA FM** ; **KARELIS AD** ; **ST-PIERRE DH** ; **GARREL D** ; **BASTARD JP** ; **TARDIF A** ; **PRUD'-HOMME D** ; **RABASA-LHORET R.** Surrogate indexes vs. euglycaemic-hyperinsulinemic clamp as an indicator of insulin resistance and cardiovascular risk factors in overweight and obese postmenopausal women. *Diabetes Metab*, 2006, vol. 32, 251-255
- **RORIZ-FILHO, J. et al.** *Biochim Biophys Acta*, 2009, vol. 1792, 432-443
- **HOLLAND PM** ; **ABRAMSON RD** ; **WATSON R** ; **GELFAND DH**. Detection of specific polymerase chain reaction product by utilizing the 5' → 3' exonuclease activity of Thermus aquaticus DNA polymerase. *Proc Natl Acad Sci USA*, 1991, vol. 88, 7276-7280
- **GIBSON UEM** ; **HEID CA** ; **WILLIAMS PM**. A Novel Method for Real Time Quantitative RT-PCR.. *Genome Res*, 1996, vol. 6, 995-1001
- **BUSTIN SA**. Absolute quantification of mRNA using real time reverse transcription polymerase chain reaction assays.. *J Mol Endocrinol*, 2000, vol. 25, 169-193
- **CAO L**. Advances in Digital Polymerase Chain Reaction (dPCR) and Its Emerging Biomedical Applications. *Biosens Bioelectron*, 2017, vol. 90, 459-474
- Strategies for Successful Gene Expression Assays. *Tech Note*, 2015
- **VELDMAN-JONES MH et al.** Evaluating Robustness and Sensitivity of the NanoString Technologies nCounter Platform to Enable Multiplexed Gene Expression Analysis of Clinical Samples. *Cancer Res*, 2015, vol. 75 (13), 2587-93
- **SABOUR L et al.** Clinical Applications of Next-Generation Sequencing in Cancer Diagnosis. *Pathol Oncol Res*, 2016, vol. 23 (2), 225-234
- **MARDIS ER**. DNA sequencing technologies: 2006-2016. *Nat Protoc.*, 2017, vol. 12 (2), 213-218
- **KAMPS R**. Next-Generation Sequencing in Oncology: Genetic Diagnosis, Risk Prediction and Cancer Classification. *Int J Mol Sci*, 2017, vol. 18 (2), 308
- **MOROZOVA O et al.** Applications of new sequencing technologies for transcriptome analysis. *Annu Rev Genomics Hum Genet*, 2009, vol. 10, 135-51

- **YADAV NK**. Next Generation Sequencing: Potential and Application in Drug Discovery. *Scientific World Journal*, 2014
- **WANG J** ; **SONG Y**. Single cell sequencing: a distinct new field. *Clin Transl Med*, 2017, vol. 6 (1), 10
- **CUEVAS-DIAZ DURAN R** ; **WEI H** ; **WU JQ**. Single-cell RNA-sequencing of the brain. *Clin Transl Med*, 2017, vol. 6 (1), 20
- **GARRIDO-CARDENAS JA** ; **GARCIA-MAROTO F** ; **ALVAREZ-BERMEJO JA** ; **MANZANO-AGU-GLIARO F**. DNA Sequencing Sensors: An Overview. *Sensors*, 2017, vol. 17, 588
- **GUPTA AK** ; **GUPTA UD**. Next Generation Sequencing and Its Applications. *Animal Biotechnology Models in Discovery and Translation*, 2014
- **HARRINGTON CT** ; **LIN EI** ; **OLSON MT** ; **ESHLE-MAN JR**. Fundamentals of pyrosequencing. *Arch Pathol Lab Med*, 2013, vol. 137 (9), 1296-303
- **HRDLICKOVA R** ; **TOLOUE M** ; **TIAN B**. RNA-Seq methods for transcriptome analysis. *Wiley Interdiscip Rev RNA*, 2017, vol. 8 (1)
- STATISTICA Data Miner. *StatSoft*, 2011
- **HASTIE T** ; **TIBSHIRANI T** ; **NARASIMHAN B** ; **CHU G.** *Imputation for microarray data.*, 2014
- **FRIEDMAN J** ; **HASTIE T** ; **TIBSHIRANI R.** Regularization Paths for Generalized Linear Models via Coordinate Descent.. *Journal of Statistical Software, 2010*, 2010, vol. 33, 1-22, http://www.jstatsoft. org/v33/i01
- **BAIR E.** ; **TIBSHIRANI R.** *Supervised principal components*, 2012, http://CRAN.R-project.org/package=superpc